# EUROPEAN PATENT SPECIFICATION

(11) **EP 3 068 396 B1**
(45) Date of publication and mention of the grant of the patent: **08.05.2019**
(21) Application number: 14860787.2
(22) Date of filing: 10.11.2014
(51) Int. Cl.: A61K 31/473, A61K 31/485, A61K 31/00, A61K 9/20, A61K 9/26, A61K 47/30, A61K 47/00, A61P 25/16, A61K 9/00, A61K 9/14, A61K 9/16, A61K 9/19

(54) **RAPIDLY DISINTEGRATING FORMULATIONS AND METHODS OF USE**
SCHNELL ZERFALLENDE FORMULIERUNGEN UND VERFAHREN ZUR VERWENDUNG
FORMULATIONS À DÉSINTÉGRATION RAPIDE ET LEURS MÉTHODES D'UTILISATION

(30) Priority: 11.11.2013 US 201361902589 P
(43) Date of publication of application: 21.09.2016
(60) Divisional application: 19165989.5
(73) Proprietor: Impax Laboratories, Inc., Hayward, CA 94544 (US)
(72) Inventor: CHIN, Shook-fong, Hayward, CA 94541 (US); DONG, Liang, Sunnyvale, CA 94086 (US); GUPTA, Suneel, Sunnyvale, CA 94087 (US)
(74) Representative: De Gregori, Antonella
(86) International application number: PCT/US2014/064833
(87) International publication number: WO 2015/070156

(56) References cited:
- WO-A1-97/06786
- WO-A1-97/06786
- WO-A1-2010/144817
- WO-A1-2011/156578
- WO-A1-2012/075473
- WO-A2-2004/075824
- WO-A2-2006/085101
- WO-A2-2008/120548
- WO-A2-2009/108828
- RU-C1- 2 283 650
- US-B1- 6 316 027

## Description

### FIELD OF THE INVENTION

The present invention as defined by the claims relates to the field of oral dosage forms and methods of using the dosage forms. More specifically, the present invention relates to the field of oral dosage forms which disintegrate rapidly in the saliva of the oral cavity. The rapidly disintegrating dosage forms allow delivery of a therapeutic agent or drug to the oral cavity of a patient which in turn improves the pharmacokinetics of therapeutic agents by accelerating onset time and avoiding or reducing the first pass metabolism of the therapeutic agent in the liver. In an embodiment of the present invention, the therapeutic agent in the dosage form is apomorphine.

### BACKGROUND OF THE INVENTION

Rapidly disintegrating oral dosage forms are known in the art. Some rapidly disintegrating oral dosage forms are described in United States Patent Nos. 4,371,516; 5,178,878; 5,298,261; 5,464,632; 5,587,180; 5,720,974; 5,807,576; 5,866,163; 5,869,098; 6,048,541; 6,149,938; 6,200,604; 6,316,029; 8,119,158; 8,454,996; and 8,470,361. These prior art rapidly disintegrating oral dosage forms employ a variety of techniques to facilitate the rapid disintegration of the dosage forms. For example, U.S. Patent Nos. 4,371,516 and 5,298,261 describe preparation of dosage forms that employ a lyophilization step; U.S. Patent Nos. 6,200,604 and 8,119,158 describe the use of effervescent couples; U.S. Patent Nos. 5,178,878 and 6,264,981 describe the use of large quantities of highly water soluble sugar alcohols; and U.S. Patent Nos. 8,454,996 and 8,470,361 describe the use of disintegrants with ordered mixtures of drug and carrier particles.

Delivery of a drug to the oral cavity of a patient can have many advantages such as better patient compliance, particularly in younger and older patients that are unable or unwilling to swallow a tablet or capsule. Delivery of a drug to the oral cavity of a patient also has a much shorter onset time, i.e., the time from administration to therapeutic effect, than a swallowed oral dosage form. A drug absorbed via the oral mucosa will also avoid first pass metabolism, in which the drug is metabolized in the GI tract and liver. This further allows sublingual delivery of a drug to yield greater bioavailability than other routes of administration (see Gancher, ST, et al. Absorption of Apomorphine by Various Routes in Parkinsonism. Mov Discord 1991; 6(3):212-6). Delivery of a drug to the oral cavity of a patient is also simple and can be administered by the caregiver or the patient with minimal discomfort and effort.

To achieve the benefits of delivery of a drug to the oral cavity of a patient, solid dosage forms must be formulated to take into account the oral cavity's unique environment that may complicate the transmucosal delivery of the drug. For example, one problem of the oral cavity is that there is relatively little solvent into which a solid dosage form can dissolve. The relative amounts of saliva produced in given circumstances can vary widely. On the average, salivary glands produce between 800 to 1500 mL saliva per day. In a resting, unstimulated state, salivary glands produce about 0.5 mL mucous-type saliva per minute, while stimulated salivary glands produce about 1 to 3 mL per minute. Therefore, during the 5 to 15 minutes required for the oral mucosal delivery of a drug from a solid dosage form, the total amount of saliva produced is about 5 to about 15 mL, which is a small volume compared to 600 to 1000 mL of potential solvent produced in the GI tract.

Additionally, there is a limited period of time during which the solid dosage form can be dissolved and absorbed. A swallowed solid dose form will remain in the GI tract for several hours while an oral cavity delivered solid dosage form typically remains in the oral cavity for a few minutes, typically 3-15 minutes. During this short time period in the oral cavity, the solid dosage form has to be disintegrated, the drug dissolved and absorbed.

Another problem encountered by an oral cavity dosage form is the limited surface area available for drug absorption. The surface area in the oral cavity is about 200 cm², which is relatively small compared to the surface area of the GI tract, which is about 350,000 cm².

A further problem that may be associated with drug delivery to the oral cavity is the pH of the oral cavity. The pH of the oral cavity typically ranges from about 6.2 to about 7.4. Many drugs may not be soluble and/or stable in this pH range creating further issues with drug delivery and absorption.

In addition to the difficulties presented by the oral cavity's unique environment, the physicochemical properties of the drug can present challenges and complications that affect absorption of a drug across the oral mucosa. The solubility, the dissolution rate, and the partition coefficient are factors that contribute to the extent to which a drug can be absorbed across the oral mucosa. Solubility and dissolution rate are important in creating a concentration gradient, which is the driving force for drug delivery.

The solubility of a drug is an inherent characteristic of the drug in a particular solvent. The relative affinities of the solute molecules and the solid phases determine the solubility. The solubility of a drug is a specific thermodynamic property, and an imbalance in a thermodynamic state will cause a change toward re-establishing a balance within the system. Because solubility is a specific thermodynamic property, any imbalance that causes a change away from solubility equilibrium will result in a change in the system toward re-establishing balance.

The dissolution rate of the drug, unlike the solubility and partition coefficient, is a kinetic property of a drug. An otherwise effective drug may have a dissolution rate which is acceptable for one delivery method but which is too slow for the particular time frame of another. For example, the dissolution rate of a drug may be acceptable for GI delivery, but the dissolution rate may not be practical for oral mucosal delivery. The time frame in oral mucosal delivery is about 1 to 15 minutes rather than 4 to 6 hours as in the GI tract.

The partition coefficient is the concentration ratio of a drug between two phases. Partition coefficient is determined largely by the inherent properties of the drug. In the case of oral mucosal delivery, the attraction of drug molecules between two phases on the solution/tissue interface determines the partition coefficient of the drug. As with solubility, partition coefficient is a thermodynamic property, and any imbalance will cause a change toward re-establishing a balanced state.

The physicochemical properties of a drug may be manipulated by changing the surrounding environment. For example, the solubility of an ionizable drug can be greatly increased by changing the pH of the solution to a value at which the drug is in its ionized form. Attempts to advantageously manipulate one particular physicochemical property can have a negative impact on another property. For instance, in designing a solid dosage form, a formulator may attempt to increase the drug absorption by manipulating pH, but the altered pH negatively impacts other aspects of the formulation, such as the partition coefficient and/or the stability of the drug and dosage form.

There are several ways to increase solubility and dissolution rate of a drug. One method is to employ a co-solvent. Many drugs that are insoluble in aqueous media are more soluble in organic solvents. Unfortunately, the use of solvents and co-solvents are not useful in the design of solid dosage forms. Some relatively insoluble drugs can be combined with other molecules to form more soluble complexes. Cyclodextrins, for example, have been used in many formulations to increase the solubility of poorly soluble, hydrophobic drugs.

Drugs that are weak acids or weak bases can react with base or acid, respectively, to form a salt. The ionized salt forms will almost always have higher solubilities and dissolution rates than the unionized forms. In many cases, the salt forms are also more stable chemically or physically. The ionized forms of the dissolved salts typically exhibit a lower partition coefficient than the unionized forms, and therefore are less well absorbed by the oral mucosa. Thus, converting the weak acid or base to an ionized form, i.e., a salt, in order to increase solubility may compromise absorption.

Buffering agents have been employed in solid dosage forms to control the pH of the microenvironment surrounding the drug and improve the solubility, dissolution and stability of a drug. A buffer system consists of hydrogen ion donor(s) (acid) and conjugate hydrogen ion receiver(s) (base). Optimizing the pH may also compromise the solubility and partition coefficient for oral mucosal drug delivery.

Many drugs, some of which are described below, may benefit from the oral mucosal delivery because the oral mucosal delivery will avoid extensive first pass metabolism, allow for a quicker absorption and thereby allow for lower doses and faster therapeutic onset. One particular class of drugs of particular interest for oral mucosal delivery is dopamine agonists, such as apomorphine in particular. Dopamine agonists, such as apomorphine, are useful in the treatment of Parkinson's disease.

Parkinson's disease is a progressive degenerative disease of the central nervous system. The risk of developing Parkinson's disease increases with age, and afflicted individuals are usually adults over 40.

Although the primary cause of Parkinson's disease is not known, it is characterized by degeneration of dopaminergic neurons of the *substantia nigra.* The *substantia nigra* is a portion of the lower brain, or brain stem that helps control voluntary movements. The shortage of dopamine in the brain caused by the loss of these neurons is believed to cause the observable disease symptoms.

The symptoms of Parkinson's disease vary from patient to patient. The most common symptom is a paucity of movement and rigidity, characterized by an increased stiffness of voluntary skeletal muscles. Additional symptoms include resting tremor, bradykinesia (slowness of movement), poor balance, and walking problems. Common secondary symptoms include depression, sleep disturbance, dizziness, stooped posture and dementia, problems with speech, breathing, and swallowing. The symptoms become progressively worse with time and ultimately result in death.

A variety of therapeutic treatments for Parkinson's disease are available such as the administration of levodopa, a dopamine precursor. While levodopa administration can result in a dramatic improvement in symptoms, patients can experience serious side-effects, including nausea and vomiting. Long term treatment with levodopa likely induces dyskinesia. Concurrent carbidopa administration with levodopa inhibits levodopa metabolism in the gut, liver and other tissues, thereby allowing more levodopa to reach the brain. Other dopamine agonists, such as bromocriptine, pergolide, pramipexole, and andropinirole are also used to treat Parkinson's disease and can be administered to patients either alone or in combination with levodopa.

Many patients develop involuntary choreiform movements which are the result of excessive activation of dopamine receptors. These movements usually affect the face and limbs and can become very severe. Such movements disappear if the dose of dopamine precursor (e.g., levodopa) or dopamine agonist is reduced, but this typically causes rigidity to return. Moreover, the margin between the beneficial and the unwanted effects appears to become progressively narrower as the period of drug treatment lengthens and the disease stage advances.

A further complication of long-term treatment with dopamine agonists is the development of rapid fluctuations in clinical state where the patient switches suddenly between mobility and immobility for periods ranging from a few minutes to a few hours. The fluctuations are of several general types. "Wearing-off" phenomena are deteriorations in the relief afforded by a dose of levodopa before the next dose takes effect (Van Laar T., CNS Drugs, 17:475 (2003)). Because they are related to a patient's dose schedule, such periods are often relatively predictable (Dewey R B Jr., Neurology, 62 (suppl 4):S3-S7 (2004)). In contrast, "on-off' phenomena are sudden transitions from an "on" period of levodopa benefit to an "off" period of akinesia, rigidity, and tremor that occurs in minutes or even seconds, (Swope D M., Neurology, 62 (suppl 4):S27-S31 (2004)) with no discernible relation to a patient's dose schedule. Two other phenomena are the delayed "on" effect, in which levodopa's effects are substantially delayed, and dose failure (also known as the no-"on" or skipped-dose effect), in which no effects occur at all. These various "off' states can produce such an abrupt loss of mobility that the patient may suddenly stop walking or be unable to rise from a chair in which he had sat down normally a few moments earlier.

Subcutaneous injections of apomorphine have proved to be effective in the treatment of "on-off' fluctuations in Parkinson's disease within 5 to 15 minutes of administration, and last for 45 to 90 minutes. Trials have shown consistent reversal of "off" period akinesia, a decrease in daily levodopa requirements and consequently a decrease in the amount of "on" period dyskinesias. Advantages over other dopamine agonists include a quick onset of action and lower incidence of psychological complications. For a "rescue therapy" in patients with "on-off" fluctuations, apomorphine also has the advantage over other dopamine agonists that it has a relatively short half-life.

Numerous formulations and routes of administration for apomorphine have been studied, and apomorphine therapy has been found to be hampered by various complications. For example, oral administration of apomorphine tablets exhibit a bioavailability of about 4% and therefore have required high doses to achieve the necessary therapeutic effect. It is believed that the low oral bioavailability is due to extensive metabolism in the small intestine and/or, upon absorption, in the liver; sublingual administration of apomorphine tablets caused severe stomatitis on prolonged use with buccal mucosal ulceration in approximately 30% of the patients treated (see Deffond et al., J. Neurol. Neurosurg. Psychiatry 56:101 (1993)); and intranasal administration produced transient nasal blockage, burning sensation and swollen nose and lips (see Koller et al., Neurology 62:S22 (2004)). While subcutaneous injections of apomorphine have proven effective, an injection by needle is difficult for Parkinson's patients because of impaired motor function. Furthermore, a common side effect of subcutaneous injection is the development of nodules, which often become infected, necessitating antibiotic treatment or surgical debridement (see Prietz et al., J. Neurol. Neurosurg. Psychiatry 65:709 (1998)).

Sublingual apomorphine solid dosage forms are described in U.S. Patent No. 5,624,677 and have been commercially sold in European markets by Takeda Pharmaceuticals under the tradename IXENSE and Abbott Laboratories under the tradename UPRIMA.

WO 97/06786 A1 discloses lyophilized apomorphin compositions which are designed to release the active ingredient rapidly (within 60 seconds) in the oral cavity for use in the treatment of Parkinson's disease.

A subcutaneous injectable form of apomorphine is commercially available in the United States under the tradename APOKYN®. Although apomorphine is most stable at an acidic pH of about 1, a pH range of 3-4 has been adopted as part of the pharmacopoeial specification for the APOKYN® product to maintain its stability. At higher pH, the rate of apomorphine oxidation is rapid, thereby resulting in unwanted drug degradation.

Apomorphine is a weak base and therefore an equilibrium will exist between the protonated drug and its free base. Although the protonated apomorphine is more stable at low pH, it is less permeable and more irritating to mucosal tissue.

Efforts to improve upon the delivery and stability of apomorphine dosage forms have been described in the art. For example, U.S. Patent No. 8,414,922 teaches a sublingual multilayer film composition wherein one layer comprises apomorphine HCl and another layer comprises a neutralizing agent. It is reported that this structure will remain stable during storage because the apomorphine HCl is in an acidic environment, but upon administration to a patient's oral cavity the neutralizing agent will increase the pH of the environment surrounding the apomorphine and enhance absorption across the mucosal tissues. U.S. Published Patent Application No. 2009/0023766 teaches a delivery kit with two compartments wherein one compartment comprises a low pH apomorphine HCl solution and the other compartment comprises a pH modifying solution. The two solutions can be combined prior to buccal administration to increase the pH of the environment surrounding the apomorphine and enhance absorption across the mucosal tissues. These prior art methods for administering apomorphine to a patient's oral cavity attempt to solve apomorphine stability/permeability issues discussed above. However, due to its lower solubility at neutral pH, apomorphine particles dispersed in the polymeric film of U.S. Patent No. 8,414,922 will dissolve slowly, or the apomorphine may precipitate after mixing the two solutions taught in U.S. Published Patent Application No. 2009/0023766. These disadvantages of the prior art apomorphine buccal administration result in a poor and/or slow bioavailability.

In light of the foregoing background, one object of the present invention is to develop stable solid dosage form that can be administered to a patient's oral cavity and allow the oral mucosal absorption of a drug.

A further object of the present invention is to develop a solid solution comprising a drug and at least one matrix polymer that can inhibit the drug precipitation and/or recrystallization during and after dilution with a neutral pH aqueous medium.

An additional object of the present invention is to develop a drug-containing solid solution/dispersion that will rapidly dissolve and/or disintegrate in saliva when placed in the oral cavity of a patient, without the need for additional water and wherein the drug, such as apomorphine, will remain solubilized in the patient's saliva, i.e., a pH range of about 6.2 to about 7.4, after solid solution has dissolved or disintegrated.

A still further object of the present invention is to develop a solid dosage form in a powder form comprising a drug containing solid solution/dispersion.

Another further object of the present invention is to develop a solid dosage form in a tablet form comprising a drug containing solid solution/dispersion.

It is another further object of the present invention to develop a stable solid dosage form that resists degradation, including but not limited to oxidation, of the drug during manufacture and upon storage for at least one month to 2 years.

A still further object of the present invention is to develop a process for preparing a solid solution/dispersion comprising a drug that is poorly water-soluble at neutral pH levels, and at least one matrix polymer that employs water and no organic solvents, while maintaining stability of the drug in base form.

A further object of the present invention is a method for treating afflictions such as Parkinson's disease and symptoms associated with the afflictions comprising: placing a rapidly dissolving or disintegrating solid dosage form into a patient's oral cavity wherein the rapidly dissolving or disintegrating solid dosage form comprises a drug containing solid solution/dispersion; allowing the rapidly dissolving disintegrating solid dosage form to dissolve or disintegrate in the saliva of the patient's oral cavity, thereby facilitating the absorption of the drug across or through the oral mucosa of the patient. The scope of the invention is defined by the claims. Any references in the description to methods of treatment refer to the compounds, pharmaceutical compositions and medicaments of the present invention for use in a method for treatment of the human (or animal) body by therapy (or for diagnosis).

### SUMMARY OF THE INVENTION

The forgoing objectives and others are met by the present invention which is a rapidly dissolving or disintegrating solid dosage form for the oral mucosal delivery of 0.1 to 100 mg of apomorphine as defined by claim 1.

Embodiments of the present invention will comprise a drug/polymer solid solution/dispersion that comprises 50 wt% or less of a therapeutic agent or drug and 50 wt% or more of a water soluble polymer. A substantial portion of the therapeutic agent or drug present in the drug/polymer solid solution/dispersion should be in the form of a free base or non-ionized form, acceptable salt forms, or mixture of free base and salt forms (ionized forms) in acceptable ratio. A substantial portion of the therapeutic agent or drug in the drug/polymer solid solution/dispersion should also be in an amorphous, or non-crystalline, form. The drug/polymer solid solution/dispersion may also comprise additional excipients such as antioxidants, pharmaceutically acceptable acids and bases, buffers, solubilizers and mixtures therefore.

The drug/polymer solid solution/dispersion may be administered to a patient's oral cavity or may be combined with additional pharmaceutical excipients for administration to a patient's oral cavity.

The solid dosage forms of the present invention, i.e., the drug/polymer solid solution/dispersion alone or combined with additional pharmaceutical excipients, should dissolve within 5 minutes or less, preferably 2.5 minutes or less and most preferably within 1 minute or less when placed in simulated saliva with a pH of 6.2± 1 and at a temperature of 37 ±0.5°C.

The solid dosage form of the present invention should also be stable during manufacture and storage. Embodiments of the present invention should prevent an increase of drug degradation products, sometimes referred as degradation impurities, during the manufacture of the dosage form, during normal storage conditions and during accelerated storage conditions. Specifically the solid dosage form should contain about 2.0% or less of any individual drug degradation product or impurity, preferably about 1.5% or less of any individual drug degradation product or impurity and preferably about 1.0 % or less of any individual drug degradation product or impurity. The solid dosage form should also contain a total drug degradation product or impurity amount of about 2.5% or less, preferably about 2.0% or less and most preferably 1.5% or less. The foregoing percentages of drug degradation products and/or impurities are based on the total amount of drug in the solid dosage form and not the total weight of the solid dosage form.

In certain embodiments of the present invention, the solid dosage form of the present invention will be placed in the oral cavity of a patient, preferably in the buccal region, sublingual region or gingival region of the patient's oral cavity to facilitate, promote and/or allow for the oral mucosal absorption of the drug. Further embodiments protect the free base form of the drug present in the drug/polymer solid solution/dispersion, such as apomorphine, from precipitation in the patient's saliva after the solid dosage form has dissolved or disintegrated in the patient's oral cavity, allow rapid drug partitioning to buccal, sublingual or gingival tissue and thereby minimize the potential of the drug being swallowed. Still further embodiments permit effective buccal, sublingual or gingival administration of the dosage form for absorption of the drug, particularly apomorphine, across the oral mucosa of the patient, thereby producing a plasma profile similar to that of a plasma profiles obtained from the subcutaneous injections of a drug-containing composition.

The solid dosage form comprises a therapeutically effective amount of apomorphine, preferably in a substantially free base form or non-ionized form and in a substantially amorphous form. This embodiment may be placed in the oral cavity of a patient, preferably in the buccal region, sublingual region or gingival region of the patient's oral cavity to facilitate, promote and/or allow for the oral mucosal absorption of the apomorphine for treating Parkinson's disease and symptoms of Parkinson's disease including hypomobility, "off" episodes ("end-of-dose wearing off") and unpredictable "on/off" episodes.

### BRIEF DESCRIPTION OF THE FIGURES

FIG. 1 depicts the dissolution profiles of the compositions prepared in Examples 1 and 2 when tested in 5 mL of simulated saliva, pH 6.2 at 37°C.
FIG. 2 depicts the plasma concentration profiles obtained from the administration of the composition prepared in Example 1 and APOKYN® to six rabbits.
FIGs. 3a-3d depict the X-ray powder diffraction patterns of compositions described in Example 17.
FIGs 4a-4d depict modulated DSC thermograms of the compositions described in Example 17.
FIG. 5a and 5b depict the pharmacokinetic profile of the dosage forms described in Example 18.

### DETAILED DESCRIPTION OF THE INVENTION

Before the present invention is further described, it is to be understood that this invention is not limited to the particular embodiments described. It is also to be understood that the terminology used herein is for the purpose of describing particular embodiments only, and is not intended to be limiting, since the scope of the present invention will be limited only by the appended claims.

As used herein and in the appended claims, the singular forms "a," "an," and "the" include plural referents unless the context clearly dictates otherwise.

The terms "individual," "subject," or "patient" are used interchangeably. As used herein, they mean any mammal. In some embodiments, the mammal is a human. In some embodiments, the mammal is a non-human.

The terms "treat," "treating," or "treatment," and other grammatical equivalents as used herein, include alleviating, inhibiting or reducing symptoms, reducing or inhibiting severity of, reducing incidence of, prophylactic treatment of, reducing or inhibiting recurrence of, preventing, delaying onset of, delaying recurrence of, abating or ameliorating a disease or condition symptoms, ameliorating the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition. The terms further include achieving a therapeutic benefit. By therapeutic benefit is meant eradication or amelioration of the underlying disorder being treated, and/or the eradication or amelioration of one or more of the physiological symptoms associated with the underlying disorder such that an improvement is observed in the individual.

The terms "effective amount" or "therapeutically effective amount" as used herein, refer to a sufficient amount of at least one therapeutic agent or drug being administered which achieves a desired result, e.g., to relieve to some extent one or more symptoms of a disease or condition being treated. In certain instances, the result is a reduction and/or alleviation of the signs, symptoms, or causes of a disease, or any other desired alteration of a biological system. In some embodiments, the effective amount is a dose that is generally effective in alleviating, reducing, noticeably reducing, or eliminating, symptoms associated with Parkinson's disease including but not limited to tremors, hypomobility, "off" episodes ("end-of-dose wearing off" and unpredictable "on/off" episodes), restless leg syndrome, sexual dysfunction and depressive disorders such as depression, bipolar disorder or mania. In certain instances, an "effective amount" for therapeutic uses is the amount of the composition comprising an agent as set forth herein required to provide a clinically significant decrease in a disease. An appropriate "effective" amount in any individual case is determined using any suitable technique, such as a dose escalation study.

The terms "administer," "administering," "administration," and the like, as used herein, refer to the methods that are used to enable delivery of agents or compositions to the desired site of biological action. These methods include, but are not limited to, the oral cavity which includes the buccal region, sublingual region and/or the gingival region of the oral cavity.

The term "pharmaceutically acceptable" as used herein, refers to a material that does not abrogate the biological activity or properties of the agents described herein, and is relatively nontoxic (i.e., the toxicity of the material is significantly outweighed by the benefit of the material). In some instances, a pharmaceutically acceptable material is administered to an individual without causing significant undesirable biological effects or significantly interacting in a deleterious manner with any of the components of the composition in which it is contained.

The term "normal storage conditions" refers to storage at room temperature, approximately 25°C and approximately 60% relative humidity for at least three months, preferably at least six months and most preferably at least one year. The solid dosage form should be stored in pharmaceutically acceptable containers such as glass bottles, plastic bottles or blister packaging with or without a desiccant.

The term "accelerated storage conditions" refers to storage at approximately 40°C and approximately 75% relative humidity for at least two weeks or longer, one month or longer, two months or longer, three months or longer, four months or longer, five months or longer and six months or longer. The solid dosage form should be stored in pharmaceutically acceptable containers such as glass bottles, plastic bottles or blister packaging with or without a desiccant.

The term "substantially" as used to describe, for example, the amount of free base or amorphous form of the drug present in the drug/polymer solid solution/dispersion means at least 5% or greater, 10% or greater, 15% or greater, 20% or greater, 25% or greater, 30% or greater, 35% or greater, 40% or greater, 45% or greater, 50% or greater, 55% or greater, 60% or greater, 65% or greater, 70% or greater, 75% or greater, 80% or greater, 85% or greater, 90% or greater, or 95% or greater.

The term "amorphous" indicates that the drug and, more particularly, the drug in the drug/polymer solid solution/dispersion lacks a definite crystal lattice structure. An X-ray Powder Diffraction Pattern of an amorphous material will lack clear, separate and distinct 2θ peaks that are characteristic of a crystalline form of the drug.

The term "solid dispersion" is intended to encompass a solid composition comprising, consisting essentially of and/or consisting of a matrix polymer and drug. The solid dispersion may be prepared by removing a solvent from a liquid composition comprising a matrix polymer in solution and a drug. The drug in the liquid composition may be in solution with the matrix polymer and/or dispersed in the liquid composition containing the matrix polymer in solution. Once the solvent is removed from the liquid composition, the resulting solid, i.e., "solid dispersion" should contain the drug uniformly and homogeneously distributed or dispersed throughout the polymer matrix.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

The publications discussed herein are provided solely for their disclosure prior to the filing date of the present application. Nothing herein is to be construed as an admission that the present invention is not entitled to antedate such publication by virtue of prior invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, the preferred methods and materials are now described. All publications describe the methods and/or materials in connection with which the publications are cited.

The therapeutic agents or drugs that may be used include but are not limited to opioid agonists (such as buprenorphine, fentanyl, alfentanil, sufentanil, lofentanil, and carfentanil), opioid antagonists (such as naloxone, naltrexone and nalbuphene), butyrophenones (such as droperidol and haloperidol); benzodiazepines (such as diazapam, midazolam, triazolam, oxazolam, and lorazepam); GABA stimulators (such as etomidate); barbiturates (such as thiopental, methohexital, thiamazol, pentobarbital, and hexabarbital); di-isopropylphenols drugs (such as diprivan); and other central nervous system-acting drugs such as levodopa, carbidopa and apomorphine. It will be appreciated that other drugs may also be utilized within the scope of the present invention either singly or in combination.

The following is a representative list of potential drugs that may be used in the present invention and the possible amounts:

| | |
|---|---|
| pentobarbital | 50-200 mg |
| thiopental | 50-500 mg |
| fentanyl | 0.05-5 mg |
| buprenorphine | 0.05-5 mg |
| alfentanil | 0.5-50 mg |
| sufentanil | 5-500 µg |
| lofentanil | 0.1-100 µg |
| carfentanil | 0.2-100 µg |
| naloxone | 0.05-5 mg |
| naltrexone | 0.05-5 mg |
| nalbuphene | 1-50 mg |
| diazepam | 1-40 mg |
| lorazepam | 1-5 mg |
| midazolam | 0.5-25 mg |
| oxazepam | 5-40 mg |
| droperidol | 1-20 mg |
| haloperidol | 0.5-10 mg |
| propanidid | 1-10 mg |
| diprivan | 5-20 mg |
| clondine | 0.1-0.5 mg |
| enalapril | 5-15 mg |
| nitroglycerin | 0.4-1.0 mg |
| propranolol | 0.1-50 mg |
| cardidopa | 10-50 mg |
| levodopa | 100-750 mg |
| albuterol | 0.5-5 mg |
| beclomethasone | 20-50 µg |
| epinephrine | 200-500 µg |
| flunisolide | 25-50 µg |
| dihydrogotamine | 0.5-10 mg |

In one embodiment, the therapeutic agent or drug is apomorphine, also known as 6aβ-aporphine-10, 11 diol. Apomorphine is a non-ergot dopamine agonist which has a high affinity for D₂, D₃ and D₄ and lower affinity for D₁, and D₅ receptors. It has the following structural formula:

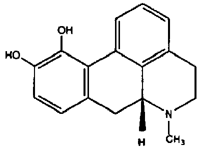

Oral doses in excess of 500 mg apomorphine have been shown to produce a dose-dependent improvement in tremor, rigidity, and akinesia but are associated with drug-induced nephrotoxicity. This is thought to be a result of nephrotoxic metabolites produced by the liver, presumably due to extensive first-pass metabolism. 2-10 mg subcutaneous injections of apomorphine have been administered to patients to treat hypomobility, "off' episodes ("end-of-dose wearing off" and unpredictable "on/off" episodes) associated with Parkinson's disease. In the present invention, the solid dosage form that is administered to the patient's oral cavity comprises 0.1 to 100 mg or about 2 to about 60 mg of apomorphine, preferably in a substantially free base form and/or a substantially amorphous form.

The amount of therapeutic agent or drug present in the drug/polymer solid solution/dispersion of the present invention should comprise about 1 wt% to about 50 wt% of the drug/polymer solid solution/dispersion, preferably about 2 wt% to about 40 wt% of the drug/polymer solid solution/dispersion and most preferably about 5 wt% to about 30 wt% of the drug polymer solid solution/dispersion.

In certain embodiments, the drug should also be present in the drug/polymer solid solution/dispersion in an amount of 5 wt% to about 100 wt% in a free base form based on the total amount of drug in the drug/polymer solid solution/dispersion, preferably about 50 wt% to about 100 wt% in a free base form based on the total amount of drug in the drug/polymer solid solution and most preferably about 60 wt% to about 100 wt% in a free base form based on the total amount of drug in the drug/polymer solid solution.

In certain embodiments, the drug should also be present in the drug/polymer solid solution/dispersion in an amount of 15 wt% to about 100 wt% in an amorphous form based on the total amount of drug in the drug/polymer solid solution, preferably about 40 wt% to about 100 wt% in an amorphous form based on the total amount of drug in the drug/polymer solid solution and most preferably about 60 wt% to about 100 wt% in an amorphous form based on the total amount of drug in the drug/polymer solid solution/dispersion.

Some embodiments of the present invention should comprise about 1 wt% to about 50 wt% of apomorphine in the drug/polymer solid solution/dispersion, preferably about 2 wt% to about 40 wt% of apomorphine in the drug/polymer solid solution/dispersion and most preferably about 5 wt% to about 30 wt% of apomorphine in the drug/polymer solid solution/dispersion. The apomorphine should also be present in an amount of 10 wt% to about 100 wt% in a free base form based on the total amount of apomorphine or apomorphine prodrug in the drug/polymer solid solution/dispersion, preferably about 40 wt% to about 100 wt% in a free base form based on the total amount of apomorphine in the drug/polymer solid solution/dispersion and most preferably about 60 wt% to about 100 wt% in a free base form based on the total amount of apomorphine in the drug/polymer solid solution/dispersion. The apomorphine may further be present in an amount of 20 wt% to about 100 wt% in an amorphous form based on the total amount of apomorphine in the drug/polymer solid solution/dispersion, preferably about 40 wt% to about 100 wt% in an amorphous form based on the total amount of apomorphine in the drug/polymer solid solution/dispersion and most preferably about 60 wt% to about 100 wt% in an amorphous form based on the total amount of apomorphine in the drug/polymer solid solution/dispersion.

The creation of the drug/polymer solid solution/dispersion of apomorphine, especially its free base form, should protect the drug from precipitation during dilution with the saliva encountered in a patient's oral cavity, especially in a pH range of about 6.2 to about 7.4 and preferably about 6.4 to about 7.2. The creation of the drug/polymer solid solution/dispersion should further permit rapid partitioning of the drug into the oral mucosa, particularly the mucosa of the buccal, sublingual and/or gingival regions. Accordingly, the administration of the drug/polymer solid solution/dispersion to a patient's oral cavity should minimize the potential of the drug being swallowed and improve the bioavailability of the drug.

If the drug/polymer solid solution/dispersion is prepared by dispersing the drug into a matrix polymer solution rather than dissolving the drug in the matrix polymer solution or mixing a drug solution with a matrix polymer solution, the particle size of the drug being dispersed should be as small as possible. The mean particle size (D₅₀) of a dispersed drug should be less than 25 microns, preferably less than 10 microns and most preferably less than 5 microns.

The matrix polymer that may be used to prepare the drug/polymer solid solution/dispersion in accordance with the present invention should be a pharmaceutically acceptable, water soluble polymer, and preferably a polymer that exhibits a viscosity of less than 500 mPa s, preferably less than 250 mPa s and most preferably less than 150 mPa s when a 10% aqueous solution is prepared. The polymer used to prepare the drug/polymer solid solution/dispersion in certain embodiments will exhibit a viscosity of less than 50 mPa s, preferably less than 25 mPa s and most preferably less than 15 mPa s when a 10% aqueous solution is prepared. Representative examples of the water soluble polymers that may be used to prepare the drug/polymer solid solution/dispersion include but are not limited to povidone, copovidone, hydroxypropyl methylcellulose, hydroxypropyl cellulose, low-substituted hydroxypropyl cellulose, polyvinyl alcohols, polyvinyl alcohol-polyethylene glycol copolymers and mixtures thereof. The amount of water soluble polymer present in the drug/polymer solid solution/dispersion of certain embodiments of the present invention should comprise about 50 to about 95 wt% of the drug/polymer solid, preferably about 65 to about 95 wt% of the drug/polymer solid and most preferably about 80 to about 95 wt% of the drug polymer solid solution.

In certain embodiments of the present invention, the polymer should exhibit a glass transition temperature significantly higher than ambient temperature, preferably at least 40 C° higher than ambient temperature, preferably at least 50 C° higher than ambient temperature and most preferably at least 60 C° higher than ambient temperature. It is understood that the presence of the drug and other excipients such as plasticizers, lubricants and surfactants may increase or decrease the glass transition temperature of the polymer or polymers used to prepare the drug polymer solid solution of the present invention. With this understanding, embodiments of the present invention should be formulated so the glass transition temperature of the polymer in the drug polymer solid solution is at least 50 C°, preferably at least 60 C° and most preferably at least 70 C°.

The drug/polymer solid solution/dispersion may also comprise additional excipients such as antioxidants, pharmaceutically acceptable acids and bases, buffers, solubilizers and mixtures therefore.

Representative antioxidants that may be used in the drug/polymer solid solution/dispersion include but are not limited to ascorbic acid, ascorbyl palmitate, butylated hydroxyanisole, butylated hydroxytoluene, hypophosphorous acid, monothioglycerol, potassium metabisulfate, propyl gallate, sodium bisulfite, sodium formaldehyde sulfoxylate, sodium metabisulfate, sodium sulfate, sodium thiosulfate, sodium dioxide, tocopherol and mixtures thereof. The amount of antioxidant present in the drug/polymer solid solution/dispersion of certain embodiments of the present invention should comprise about 0 to about 15 wt% of the drug/polymer solid, preferably about 0.05 to about 10 wt% of the drug/polymer solid and most preferably about 0.5 to about 5 wt% of the drug polymer solid.

The drug/polymer solid solution/dispersion may also contain a pharmaceutically acceptable acid or base. The pharmaceutically acceptable acid or base should be present in an amount sufficient to create and/or maintain the desired amount of the drug in the free base form. Representative examples of pharmaceutically acceptable acids that may be used include but are not limited to acetic acid, citric acid, fumaric acid, hydrochloric acid, malic acid, nitric acid, phosphoric acid, propionic acid, sulfuric acid, tartaric acid and mixtures thereof. Representative examples of pharmaceutically acceptable bases that may be used include but are not limited to ammonia, ammonium carbonate, diethanolamine, potassium hydroxide, sodium bicarbonate, sodium carbonate, sodium hydroxide, trolamine and mixtures thereof. The amount of pharmaceutically acceptable acid or base sufficient to create and/or maintain the drug in the desired amount of free base form will depend on the amount and type of drug employed in the drug/polymer solid solution. For example, if the drug is an acid addition salt, a sufficient molar quantity of a base such as sodium or potassium hydroxide may be added to the liquid composition that forms the drug/polymer solid solution/dispersion to neutralize all or part of the acid addition drug salt and create the free base drug form upon removal of the solvent to create the drug/polymer solid solution/dispersion. For example, a molar ratio of about 0.5 moles of base to about 2.0 moles of base can be added for each mole of drug present as an acid addition salt, preferably about 0.65 moles of base to about 1.5 moles of base for each mole of drug present as an acid addition salt and most preferably about 0.75 moles of base to about 1.2 moles of base for each mole of drug present as an acid addition salt.

In one embodiment of the present invention, when the drug is apomorphine hydrochloride, the liquid composition used to prepare the drug/polymer solid solution/dispersion will comprise about 0.5 moles base to about 2.0 moles of base for each mole of apomorphine hydrochloride, preferably about 0.65 moles of base to about 1.5 moles of base for each mole of apomorphine hydrochloride and most preferably about 0.75 moles of base to about 1.2 moles of base for each mole of apomorphine hydrochloride. In another embodiment of the invention, the amount of base added to the liquid composition used to prepare the drug/polymer solid solution/dispersion will range from about 0.50 moles to about 0.95 moles of base for each mole of apomorphine hydrochloride, preferably about 0.60 moles to about 0.92 mole of base for each mole of apomorphine hydrochloride and most preferably about 0.65 to about 0.90 moles of base for each mole of apomorphine hydrochloride. The preferred base is a strong base such as alkali metal or alkaline earth metal hydroxides. Representative strong bases include but are not limited to lithium hydroxide, sodium hydroxide, potassium hydroxide, calcium hydroxide or mixtures thereof.

Not all of the apomorphine hydrochloride will be neutralized with an added base during the manufacture of the drug/polymer solid solution/dispersion because it is desired that some of the apomorphine be present in the drug/polymer solution in a protonated form. Preferably the drug/polymer solid solution will comprise about 50% or less of protonated apomorphine, preferably about 40% or less of protonated apomorphine and most preferably about 35% or less of protonated apomorphine based upon the total amount of apomorphine present in the drug/polymer solid solution. The amount of protonated/non-protonated form of apomorphine in the drug/polymer solid solution/dispersion can be determined by any commonly known analytical techniques, including but not limited to proton NMR.

The drug/polymer solid solution/dispersion may also contain a buffer. Representative buffers include but are not limited to acetic acid, adipic acid, ammonium carbonate, ammonium phosphate, boric acid, citric acid, lactic acid, phosphoric acid, potassium citrate, potassium metaphosphate, potassium phosphate, sodium acetate, sodium citrate, sodium lactate, sodium phosphate, succinic acid and mixtures thereof. The amount of buffer will depend upon the drug employed in the drug/polymer solid solution and can be easily determined by reference to conventional pharmaceutical reference books such as Remington's The Science and Practice of Pharmacy, 21st ed. (2005) and the Handbook of Pharmaceutical Excipients, 5th ed. (2006).

The drug/polymer solid solution/dispersion may also contain a solubilizer. Representative examples of solubilizer include but are not limited to ionic surfactants, nonionic surfactants, polysorbates, derivatives of tocopherol, poloxamers, monoglycerides, diglycerides, fatty acids, fatty alcohols and mixtures thereof. A more complete listing of solubilizers can be found on page 3258 of the United States Pharmacopeia 29 (2006). The amount of solubilizer can range from 0 wt% to about 5 wt%.

The drug/polymer solid solution/dispersion of the present invention can be prepared by any method commonly known in the art and typically requires preparing a solution of the polymer and dissolving or dispersing the drug and any additional excipients as discussed above in the polymer solution and removing the solvent. The solvent may be removed by evaporation or drying techniques such as oven evaporation, vacuum drying, spray drying or freeze drying (sometimes referred to as lyophilization). Examples of some of the drying techniques are provided in U.S. Patent Nos. 4,371,516; 5,298,261; and 6,262,981.

In some embodiments of the present invention the drug/polymer solid solution/ dispersion should exhibit a moisture content of less than 15%, preferably less than 12% and most preferably less than 10%. In further embodiments wherein the drug/polymer solid solution/dispersion is in the form of a tablet, the moisture content should range from about 1.0% to about 10%, preferably about 1.5% to about 8.5% and most preferably about 2% to about 8%. If the drug/polymer solid solution or dispersion is in the form of a powder or granules, the moisture content may range from 0% to about 10%, preferably about 0.25% to about 8.5% and most preferably about 0.5% to about 8%. The moisture content can be measured by any technique commonly known in the industry or approved by the United States Pharmacopeia such as Karl Fisher titration.

In one embodiment of the present invention, the drug and at least one water soluble polymer are dissolved in an appropriate solvent, such as water, an organic solvent such as ethanol, acetone, isopropyl alcohol, or mixtures of water and an organic solvent(s), along with the additional excipients if necessary, and the solvent is removed to create the drug/polymer solid solution/dispersion. In certain embodiments, the solvent is water and no organic solvents are employed in preparation of the drug/polymer solid solution/dispersion.

In certain embodiments of the present invention, the polymer for the drug/polymer solid solution/dispersion should be present in an amount and type to create a super saturation environment wherein the drug, preferably a hydrophobic drug, such as apomorphine free base, will not precipitate (i.e. remained solubilized) upon dilution with saliva in a patient's oral cavity. In this embodiment, the polymer acts as a precipitate inhibitor. Examples of polymers that may be used in this embodiment are described above and preferably include but are not limited to copovidone, povidone, poloxamers and mixtures thereof. The amount of polymer employed in the this embodiment is at least 60 wt% or more, preferably at least 65 wt% or more and most preferably at least 70 wt% or more based upon the total weight of the drug/polymer solid. In an alternative embodiment the amount of polymer is at about 50 wt% to about 95 wt%, preferably about 65 wt% to about 95 wt% and most preferably about 80 wt% to about 95 wt% based upon the total weight of the drug/polymer solid.

During the process of preparing the drug/polymer solid solution/dispersion in accordance with an embodiment of the present invention wherein a salt form of the drug is neutralized in-part, to form a neutral or free base form of the drug prior to removal of the solvent from the polymer solution and drug mixture, the ratio of polymer to drug should be at a level that prevents precipitation of the drug from the polymer solution and drug mixture during neutralization with an acidifying or alkalizing composition or solution. In this embodiment that employs a neutralization step of the drug present in the polymer solution, the weight ratio of polymer to drug should be about 1:1 to about 20:1, preferably about 3:1 to about 15:1 and most preferably about 5:1 to about 10:1.

The solid dosage form of the present invention should also be stable during manufacture and storage. Embodiments of the present invention should prevent an increase of drug degradation products and/or impurities, during the manufacture of the dosage form, during normal storage conditions and accelerated storage conditions. In embodiments of the present invention comprising apomorphine and pharmaceutically acceptable salts thereof such as apomorphine hydrochloride, the possible degradation products and/or impurities include:
apocodeine (European Pharmacopeia impurity A): 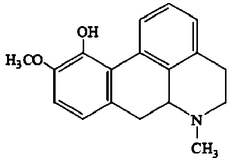
morphine (European Pharmacopeia impurity B): 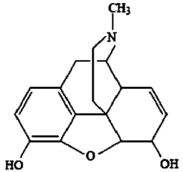
apomorphine orthoquinione: 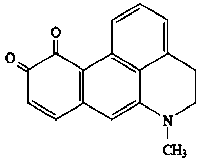 and;
apomorphine paraquinone:

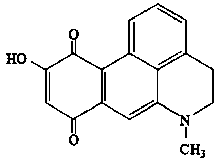

The apomorphine/polymer solid solution/dispersion embodiments of the present invention should contain about 2.0% or less of any of the above identified individual degradation products and/or impurities, preferably about 1.5% or less of any of the above identified individual degradation products and/or impurities and preferably about 1.0 % or less of any of the above identified individual degradation products and/or impurities when stored at approximately 25°C and approximately 60% relative humidity for at least three months, preferably at least six months and most preferably at least one year and/or at approximately 40°C and approximately 75% relative humidity for one month, two months, or three months. The apomorphine/polymer solid solution/dispersion embodiments of the present invention should exhibit the afore described individual degradation product levels when stored in pharmaceutically acceptable containers such as glass bottles, plastic bottles or blister packaging with or without a desiccant.

The apomorphine/polymer solid solution/dispersion embodiments of the present invention should also contain a total degradation product and/or impurity amount of about 2.5% or less, preferably about 2.0% or less and most preferably 1.5% or less when stored at approximately 25°C and approximately 60% relative humidity for at least three months, preferably at least six months and most preferably at least one year and/or at approximately 40°C and approximately 75% relative humidity for one month, two months, or three months. The apomorphine/polymer solid solution/dispersion embodiments of the present invention should exhibit the afore described total degradation product levels when stored in pharmaceutically acceptable containers such as glass bottles, plastic bottles or blister packaging with or without a desiccant.

The foregoing percentages of individual and total degradation products and/or impurities are based on the total amount of apomorphine or pharmaceutically acceptable salt thereof in the solid dosage form. The amount of individual and total degradation products and/or impurities can be determined by any methodology commonly used in the pharmaceutical arts for example by high performance liquid chromatography (HPLC).

The drug/polymer solid solution is administered to the oral cavity of a patient, for example in the form of a film or tablet placed into the buccal, sublingual or gingival regions of a patient's oral cavity or in the form of a powder that can be sprinkled or sprayed into the buccal, sublingual or gingival regions of a patient's oral cavity. The drug/polymer solid solution may be further combined with known excipients such as fillers, binders, disintegrants, sweeteners, flavoring agents, pH adjusting agents, solubilizing agent, lubricants, glidants and mixtures thereof, and the combination of drug/polymer solid solution/dispersion and excipients compressed into a tablet, disc or wafer that may be placed into the buccal, sublingual or gingival regions of a patient's oral cavity, or combination of drug/polymer solid solution/dispersion and excipients maintained or formed into a powder or granulate that may be placed into the buccal, sublingual or gingival regions of a patient's oral cavity.

Examples of fillers that may optionally be used to prepare the combination of drug/polymer solid solution/dispersion and excipients and formed into a tablet, disc, wafer, powder or granulate include but are not limited to lactose, starch, dextrose, sucrose, fructose, maltose, mannitol, sorbitol, kaolin, microcrystalline cellulose, powdered cellulose or any combination of the foregoing. In a preferred embodiment of the present invention, the filler consists of a mixture of water soluble fillers to reduce the chance of unpleasant grittiness when the tablet dissolves in the oral cavity of the patient. Most preferably, the filler will be a direct compression sugar such as confectioner's sugar, dextrates, dextrin, dextrose, fructose, maltose, mannitol, polydextrose, sorbitol, or other sugars and sugar derivatives. The amount of filler in the combination of drug/polymer solid solution and excipients and formed into a tablet, disc, wafer, powder or granulate can range from 0 wt% to about 60 wt%, preferably about 1 wt% to about 50 wt% and most preferably about 5 wt% to about 40 wt%.

Binders that may optionally be used to prepare the combination of drug/polymer solid solution and excipients and formed into a tablet, disc, wafer, powder or granulate may be one or more of the many known pharmaceutically acceptable binders. Examples of binders that may be used include but are not limited to polyvinyl alcohol, polyvinylpyrrolidone, methylcellulose, hydroxypropyl cellulose, hydroxymethyl cellulose, hydroxypropyl methylcellulose, ethylcellulose, polyacrylates and combination of the foregoing. Preferably the binder is a water soluble material. The amount of binder in the combination of drug/polymer solid solution and excipients and formed into a tablet, disc, wafer, powder or granulate can range from 0 wt% to about 20 wt%, preferably about 0.5 wt% to about 10 wt% and most preferably about 1 wt% to about 5 wt%.

Examples of disintegrants that may optionally be used to prepare the combination of drug/polymer solid solution and excipients and formed into a tablet, disc, wafer, powder or granulate include but are not limited to corn starch, croscarmellose sodium, crospovidone (polyplasdone XL-10), sodium starch glycolate (EXPLOTAB® or PRIMOJEL®) or any combination of the foregoing. The preferred disintegrants are crospovidone or sodium starch glycolate. The disintegrant may also be an effervescent agent, i.e., a compound that evolves gas by means of a chemical reaction when exposed to water or saliva. The effervescent agent typically comprises an acid source and a source of carbon dioxide. The acid source can be any of the pharmaceutically acceptable acids discussed above. The carbon dioxide source includes but is not limited to carbonate and bicarbonate salts, such as sodium bicarbonate, sodium carbonate, potassium bicarbonate, potassium carbonate, magnesium carbonate and mixtures thereof. The amount of disintegrant in the combination of drug/polymer solid solution and excipients and formed into a tablet, disc, wafer, powder or granulate can range from 0 wt% to about 50 wt%, preferably about 0.5 wt% to about 30 wt% and most preferably about 1 wt% to about 20 wt%.

The flavoring agents optionally added to the combination of drug/polymer solid solution and excipients and formed into a tablet, disc, wafer, powder or granulate preferably are taste enhancing agents and can include artificial sweeteners such as aspartame, saccharin, sucralose, dipotassium glycyrrhizinate, stevia, thaumatin and flavorants such as citric acid, peppermint oil, wintergreen oil, menthol, lemon, lime, orange grape, cherry and vanilla extract. Additional taste enhancing agents are described in U.S. Patent No. 6,027,746. In a preferred embodiment of the present invention, the flavoring agent is preferably a taste enhancing agent and may comprise a mixture of artificial sweeteners and flavorants such as aspartame and peppermint oil or grape extract.

The pH adjusting agents that may optionally be used in the combination of drug/polymer solid solution and excipients and formed into a tablet, disc, wafer, powder or granulate can be any of the above described pharmaceutically acceptable acids, bases, buffering agents or mixtures thereof. The pH adjusting agents may further aid in adjusting the pH of the buccal, sublingual or gingival regions of the oral cavity to enhance or maintain a desired pH environment for absorption of the drug and to further prevent the drug from precipitating once it is released from the administered dosage form. The amount of pH adjusting agent should be used in an amount sufficient to produce a desired pH range in the oral cavity of the patient but less than an amount which would cause adverse effects such as irritation or drug precipitation.

The solubilizing agent or solubilizer that may optionally be used in the combination of drug/polymer solid solution/dispersion and excipients and formed into a tablet, disc, wafer, powder or granulate are also described above. The amount of solubilizing agent in the combination of drug/polymer solid solution and excipients and formed into a tablet, disc, wafer, powder or granulate can range from 0 wt% to about 15 wt%, preferably about 0.01 wt% to about 10 wt% and most preferably about 0.05 wt% to about 5 wt%.

Examples of lubricants that may optionally be used in the combination of drug/polymer solid solution/dispersion and excipients and formed into a tablet, disc, wafer, powder or granulate include but are not limited to calcium stearate, glyceryl behenate, magnesium stearate, mineral oil, polyethylene glycol, castor oil, hydrogenated castor oil, vegetable oil, sodium stearyl fumarate, zinc stearate and mixtures thereof. The amount of lubricant in the combination of drug/polymer solid solution and excipients and formed into a tablet, disc, wafer, powder or granulate can range from 0 wt% to about 5 wt%, preferably about 0.01 wt% to about 3 wt% and most preferably about 0.5 wt% to about 2 wt%.

Examples of glidants that may optionally be used in the combination of drug/polymer solid solution and excipients and formed into a tablet, disc, wafer, powder or granulate include but are not limited to talc, colloidal silicon dioxide, magnesium silicate, calcium silicate and mixtures thereof. The amount of glidant in the combination of drug/polymer solid solution and excipients and formed into a tablet, disc, wafer, powder or granulate can range from 0 wt% to about 5 wt%, preferably about 0.01 wt% to about 3 wt% and most preferably about 0.5 wt% to about 2 wt%.

The drug/polymer solid solution that is administered to the oral cavity of a patient, preferably in the form of a film, tablet, disc wafer, powder or granulate, should dissolve within 5 minutes or less, preferably 2.5 minutes of less and most preferably within 1 minute or less when placed in 5 mL of simulated saliva with a pH of 6.2± 1 and at a temperature of 37 ±0.5°C.

The drug/polymer solid solution/dispersion that is administered to the oral cavity of a patient, preferably in the form of a film, tablet, disc, wafer, powder or granulate should release the drug in a rapid manner so that at least 5% of the drug, preferably at least 10% of the drug and most preferably at least 20% of the drug is absorbed across the patient's oral mucosa within 3 minutes of being placed in the patient's oral cavity, preferably within 2 minutes of being placed in the patient's oral cavity and most preferably within 1 minute of being placed in the patient's oral cavity.

The drug/polymer solid solution that is administered to the oral cavity of a patient, preferably in the form of a film, tablet, disc, wafer, powder or granulate should release the drug so that a drug plasma level, preferably a therapeutic drug plasma level is detectable within 15 minutes of placing the composition in the patient's oral cavity, preferably within 10 minutes of placing the composition in the patient's oral cavity and most preferably within 7 minutes of placing the composition in the patient's oral cavity.

The drug/polymer solid solution/dispersion that is administered to the oral cavity of a patient, preferably in the form of a film, tablet, disc, wafer, powder or granulate should also produce a mean time to maximum drug plasma level (Tₘₐₓ) of about 5 to about 45 minutes, preferably about 7.5 to about 30 minutes and most preferably about 10 to about 25 minutes based upon a single dose administration to at least six patients under fed or fasting conditions.

In certain embodiments, the amount of apomorphine administered to the oral cavity of the patient, preferably in the form of a film, tablet, disc, wafer, powder or granulate should also produce a maximum apomorphine plasma level of less than about 100 ng/mL, preferably less than about 50 ng/mL and most preferably less than about 25 ng/mL In an alternative embodiment, the target plasma level of S-ketamine should range between about 1 ng/mL and about 100 ng/mL, preferably between about 2 ng/mL and about 50 ng/mL and most preferably between about 3 ng/mL and 25 ng/mL. The foregoing plasma levels may be determined based upon a single dose fasting study in healthy volunteers or in patients.

The following examples illustrate the present invention and are not intended to limit the scope of the present invention.

### EXAMPLE 1

An apomorphine solid solution/dispersion in accordance with the present invention was prepared as follows.

0.1 g of sodium metabisulfite, 1 gm of apomorphine hydrochloride and 5.5 gm of copovidone (VA-64) were dissolved in 6 mL of purified water in a container, resulting in the apomorphine solution.

5N sodium hydroxide solution was prepared by 1 to 1 dilution from sodium hydroxide (NaOH) solution, 10N (Fisher Scientific, SS255-1). The sodium hydroxide solution (∼5N) was then added dropwise using a micropipette into the apomorphine solution while stirring. Alternatively, 1 N NaOH solution or other suitable concentrations of an NaOH can be used instead of 5 N. The solid content of the mixed solution is ∼10% by weight. After titration, the apomorphine remained solubilized in the presence of copovidone. The mixed solution with pH above 6.5 was loaded into vials and lyophilized by freezing the solution at -50°C for 4 hours, with additional freezing at -55°C for 3 hours, using AdVantage Plus EL Freeze Dryer followed by the following conditions:

| **Step#** | **Temperature** (C°) | **Time (min)** | **R/H** | **Vacuum** |
|---|---|---|---|---|
| 1 | -55 | 180 | H | 400 mTorr |
| 2 | -25 | 120 | H | 300 mTorr |
| 3 | 10 | 400 | H | 300 mTorr |
| 4 | 0 | 120 | R | 100 mTorr |
| 5 | 0 | 60 | H | 0 mTorr |
| 6 | 25 | 90 | H | 100 mTorr |
| 7 | 40 | 90 | H | 100 mTorr |
| 8 | 25 | 90 | H | 100 mTorr |

| | | | | |
|---|---|---|---|---|
| (temperature in C°; time in minutes; R is the ramp value or temperature from one set point to another for the duration of the designated period of time ; H is the hold temperature ; Vacuum in mTorr inside the lyophilizer chamber) | | | | |

After lyophilization, the lyocake was milled manually by passing it through a sieve (No. 140). The milled powders can be sprinkled directly into sublingual or buccal area of a patient or incorporated into a powder dispenser for sublingual or buccal dosing.

The resulting drug/polymer solid solution/dispersion prepared in this example had the following composition:

| **Ingredient** | **% w/w** | **g/batch** |
|---|---|---|
| Apomorphine hydrochloride | 14.90 | 1.00 |
| Sodium metabisulfite | 1.49 | 0.10 |
| Sodium hydroxide | 1.64 | 0.11 |
| Copovidone, NF (KOLLIDON VA 64) | 81.97 | 5.50 |
| Purified Water, USP** | N/A | 66.5 |
| **Total (solids)** | 100.00 | 73.2 |

| | | |
|---|---|---|
| ** Lost after freeze drying. | | |

### EXAMPLE 2

The procedure described in Example 1 was repeated in this example except that no Copovidone (Kollidone VA 64) was employed. The composition of this example was not in accordance with the present invention.

### EXAMPLE 3

The solid solution/dispersion powder formulation of Example 1 was tested for dissolution in simulated saliva (pH 6.2± 1.0) and compared to the dissolution in simulated saliva (pH 6.2± 1.0) of the powder formulation prepared in Example 2.

The following procedure was employed to obtain the dissolution profiles.
1. Pre-warm 5-mL of a dissolution medium (simulated saliva, pH 6.2± 1.0) in a glass vial at 37°± 0.5°C. Prepare duplicate samples per time-point.
2. Add the powder formulation (comprising 5 mg of apomorphine HCl, USP) either from Example 1 or Example 2 into the dissolution medium from Step 1, and gently swirl the medium to allow the powder to dissolve into the solution.
3. At specific time points, transfer the solution from Step 2 into the barrel of a 10-mL syringe with a 0.22-µm filter attached to the tip of the syringe, push the solution through the filter, and collect the filtrate for apomorphine concentration assay. The apomorphine concentration was analyzed by an HPLC method such as described below in Example 13.

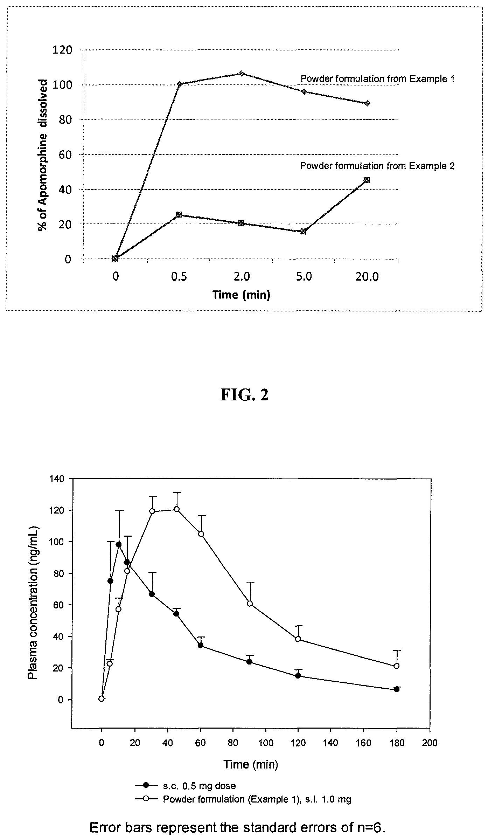

As shown in FIG.1, almost all apomorphine was solubilized in less than 1 min for the formulation prepared in Example 1 (the copovidone-comprising powder formulation), while only about 25% of the drug dissolved from the powder formulation prepared in Example 2 (without copovidone).

### EXAMPLE 4

A pharmacokinetic study was conducted with rabbits, n=6 per group. The animals were administered (under anesthesia) either the control formulation which is a subcutaneous injection of the commercially available APOKYN® composition, at a dose of 0.5 mg/animal or the oral cavity administration. The rabbits were lightly anesthetized with ketamine/zylazine (and/or isoflurane/O2 if needed). The rabbit's head was gently retrained in a fixed upright position during dosing until ∼30 sec after the drug administration. Powder formulation was sprinkled onto sublingual area of the animal's oral cavity). The powder formulation prepared in Example 1 was given at a dose of 1.0 mg/animal. At specified time points, blood was collected for plasma concentration analysis using LC/MS/MS. FIG 2 shows the apomorphine plasma concentration profiles. The bioavailability for the oral mucosal administration of the powder formulation prepared in Example 1 is 89% relative to subcutaneous administration of the APOKYN®.

### EXAMPLE 5

The preparation procedure of Example 1 is repeated in this example except with larger production batch size (∼30-fold bigger scale than Example 1), and the pre-lyophilization solution was loaded into stainless trays instead of vials for lyophilization.

The apomorphine solid solution/dispersion was prepared as follows:
3.0 g of sodium metabisulfite, 30.0 gm of apomorphine hydrochloride and 165.0 gm of copovidone (VA-64) were dissolved in 1997.0 gm of purified water in a container, resulting in the apomorphine solution.

2.9 g of sodium hydroxide was dissolved in 29.0 gm of purified water in a container, resulting in the sodium hydroxide solution (∼2.5N). The sodium hydroxide solution was then added dropwise using a transfer pipet into the apomorphine solution while stirring. The solid content of the mixed solution is ∼10% by weight. This mixed solution remained clear with no drug or polymer precipitation after titration with end point at pH above 6.5.

The solution was lyophilized in the stainless steel trays at the conditions shown in the following table after initially cooling the solution to about -60°C at a rate of 0.15 C°/min and maintaining the solution at -55°C for 5 hours, table below shown the thermal treatment steps using lyophilizer:

| **Step #** | **Temperature** | **Time** | ***R/H** |
|---|---|---|---|
| 1. | 5 °C | 30mins | H |
| 2. | -60°C | 420 mins | R |
| 3. | -55°C | 120 mins | H |

Additional set points of the lyophilizer are listed below:
(i) Freeze: -55°C;
(ii) Condenser: -70°C;
(iii)Vacuum: 13332,2 Pa (100 mTorr); and
(iv)Additional Time: 180 minutes.

**Drying cycle:**

| **Step #** | **Temperature** | **Time** | **R/H** | **Vacuum** |
|---|---|---|---|---|
| 1. | -30°C | 180 mins | H | 200 mTorr |
| 2. | -5°C | 120 mins | H | 100 mTorr |
| 3. | 10°C | 460 mins | H | 100 mTorr |
| 4. | 15°C | 270 mins | H | 100 mTorr |
| 5. | 0°C | 180 mins | R | 100 mTorr |
| 6. | 0°C | 45 mins | H | 100 mTorr |
| 7. | 20°C2 | 240 mins | R | 100 mTorr |
| 8. | 25°C | 120 mins | H | 100 mTorr |
| 9. | 30°C | 400 mins | H | 100 mTorr |

The resulting drug/polymer solid solution/dispersion prepared in this example had the following composition:

| **Ingredient** | **% w/w** | **Batch/g** |
|---|---|---|
| Apomorphine hydrochloride | 14.93 | 30.0 |
| Sodium metabisulfite | 1.49 | 3.0 |
| Sodium hydroxide | 1.43 | 2.9 |
| Copovidone, NF (KOLLIDON VA 64) | 82.15 | 165.0 |
| Purified Water, USP** | N/A | 2026.0 |
| **Total** | 100.00 | 2226.9 |
| **Total (Solid)** | | 200.9 |

| | | |
|---|---|---|
| ** Lost after freeze drying. | | |

### EXAMPLE 6

Rapid orally disintegrable/dissolving tablets may be prepared by placing the pre-lyophilization solution prepared in Example 5 into preformed blister pockets of a blister package base sheet. The filled blister package base sheet can be loaded into a freeze-dryer and lyophilized following a procedure similar to that described in Example 5.

Lyophilization (freeze-drying cycles) using blister cards may be adjusted according to the dimension of the blister cavities (i.e. the exposed surface areas, and thickness of the tablets will affects the rate of drying), and the blister cards material of constructions (i.e. material with better heat conduction/transfer will dry faster). Table below shows a typical drying cycle for aluminum blister cards with ∼19 mm diameter, and ∼1-mL volume.

The pre-lyophilization solution can be either frozen with liquid nitrogen, or using the freezing mode of a lyophilizer. Table below shown the steps to freeze the solution to -60°C using lyophilizer (i.e. Heat Treatment Step):

| **Step #** | **Temperature** | **Time** | ***R/H** |
|---|---|---|---|
| 4. | 10 °C | 30mins | H |
| 5. | -60°C | 140 mins | R |
| 6. | -60°C | 240 mins | H |

Additional set points of the lyophilizer are listed below:
(i)Freeze: -60°C;
(ii)Condenser: -70°C;
(iii)Vacuum: 26664,5 Pa (200 mTorr); and
(vi)Additional Time: 60 minutes

After freezing, the frozen samples are subjected to freeze drying with the following conditions:

| **Step #** | **Temperature** | **Time** | **R/H** | **Vacuum** |
|---|---|---|---|---|
| 1. | -30°C | 1140 mins | H | 200 mTorr |
| 2. | 10°C | 120 mins | R | 200 mTorr |
| 3. | 10°C | 120 mins | H | 200 mTorr |

After lyophilization, the blister package base sheet can be sealed by a lidding material made of an aluminum foil laminate.

The resulting rapid orally disintegrable/dissolving tablet prepared in this example may have the following composition:

| **Ingredient** | **% w/w** |
|---|---|
| Apomorphine hydrochloride | 14.93 |
| Sodium metabisulfite | 1.49 |
| Sodium hydroxide | 1.43 |
| Copovidone, NF (KOLLIDON VA 64) | 82.15 |
| **Total (solids)** | 100.00 |

### EXAMPLE 7

Rapid orally disintegrable/dissolving tablets may be prepared according to the procedure of Example 6 except the solid content of the pre-lyophilization solution before lyophilization can be varied to adjust the dose strength of the tablets. For example a 3.75%, 5%, 7.5%, 10% or higher solid content pre-lyophilization solution may be used to prepare orally disintegrable/dissolving tablets in accordance with the present invention. In addition to the adjustment for dose strength, the solid content of the pre-lyophilization solution may also be adjusted to obtain a desired tablet density, i.e. lower solid content will produce a lighter density tablet or to obtain a desired disintegration rate of the tablet.

Examples of rapid orally disintegrable/dissolving tablets that may be prepared in accordance with the present invention by varying the solid content of the pre-lyophilization solution are shown in the following table:

| Ingredients | % (w/w) | Wt/batch (g) | Wt/batch (g) | Wt/batch (g) | Wt/batch (g) |
|---|---|---|---|---|---|
| | | 3.75% solid content | 5.0% solid content | 7.5% solid content | 10.0% solid content |
| Apomorphine Hydrochloride, USP | 13.80 | 6.90 | 6.90 | 6.90 | 6.90 |
| Sodium Metabisulfite, NF | 1.38 | 0.69 | 0.69 | 0.69 | 0.69 |
| Sodium Hydroxide, NF | 1.32 | 0.66 | 0.66 | 0.66 | 0.66 |
| Copovidone, NF (Kollidon VA 64) | 82.81 | 41.40 | 41.40 | 41.40 | 41.40 |
| Polysorbate 80, NF (Tween 80) | 0.19 | 0.10 | 0.10 | 0.10 | 0.10 |
| Sucralose, NF | 0.50 | 0.25 | 0.25 | 0.25 | 0.25 |
| Purified Water, USP ** | N/A | 1283.3 | 950.0 | 616.67 | 450.0 |
| Total (Solids) | 100.00 | 50.00 | 50.0 | 50.0 | 50.0 |
| Total (include liquid)/Filling wt. | | 1333.3 | 1000.0 | 666.7 | 450.0 |

| | | | | | |
|---|---|---|---|---|---|
| ** Lost after freeze drying. | | | | | |

Below is a representative example of a 5% solid content pre-lyophilization solution without a sweetener, sucralose, and Polysorbate 80.

| **Ingredient** | **% w/w** | **Batch/g 5% solid content** |
|---|---|---|
| Apomorphine hydrochloride | 14.93 | 15.0 |
| Sodium metabisulfite | 1.49 | 1.5 |
| Sodium hydroxide | 1.43 | 1.4 |
| Copovidone, NF (KOLLIDON VA 64) | 82.15 | 82.5 |
| Purified Water, USP** | N/A | 2026.0 |
| **Total (solids)** | 100.00 | 100.45 |

| | | |
|---|---|---|
| ** Lost after freeze drying. | | |

The Polysorbate 80 can function as a processing aid in compositions prepared in accordance with the present invention. Specifically, the Polysorbate 80 will enable an easier release of the tablets from the blister cavities following lyophilization. Other polysorbates and surfactants could be substituted for the Polysorbate 80 to impart similar release properties.

### EXAMPLE 8

Rapid orally disintegrable/dissolving tablets can be prepared according to the procedure of Example 6 except the pre-lyophilization solution may further comprise a surfactant, such as Tween 80.

The resulting rapid orally disintegrable/dissolving tablet can be prepared in this example had the following composition:

| **Ingredient** | **% w/w** |
|---|---|
| Apomorphine hydrochloride | 14.3 |
| Sodium metabisulfite | 1.43 |
| Sodium hydroxide | 1.37 |
| Copovidone, NF (KOLLIDON VA 64) | 79.9 to 82.8 |
| Tween 80 | 0.1 to 3.0 |
| **Total (solids)** | 100.00 |

### EXAMPLE 9

Rapid orally disintegrable/dissolving tablets can be prepared according to the procedure of Example 6 except the molar ratio of NaOH: apomorphine that may be used are as follows:
0.2:1
0.4:1
0.5:1
0.75:1
0.85:1
0.95:1
1:1
1.2:1
1.3:1
1.4:1
1.5:1

### EXAMPLE 10

Rapid orally disintegrable/dissolving tablets were prepared according to the procedure of Example 6 except the following polymers were used instead of copovidone:
- Povidone (K30);
- Poloxamer (Lutrol® 127 micro/ Kolliphor™ P407 micro).

### EXAMPLE 11

Rapid orally disintegrable/dissolving tablets can be prepared according to the procedure of Example 6 except the following polymers may be used instead of copovidone:
- Polyvinyl alcohol, polyethylene glycol copolymer (KOLLICOAT® IR)
- Hydroxypropyl methylcellulose (METHOCEL® F50 Premium; METHOCEL® E3 Premium LV, METHOCEL® E5 Premium LV, METHOCEL® E6 Premium LV, METHOCEL® E15 Premium LV and METHOCEL® E50 Premium LV)
- Hydroxypropyl cellulose (KLUCEL® JF, KLUCEL® LF, KLUCEL® EF)

### EXAMPLE 12

Rapid orally disintegrable/dissolving tablet were prepared according to the procedure of Examples 5 and 6 with the following composition:

| **Ingredient** | **Amount per batch of 5 mg tablets** | **Amount per 5 mg tablet** | **Amount per batch of 10 mg tablets** | **Amount per 10 mg tablet** | **% w/w** |
|---|---|---|---|---|---|
| Apomorphine hydrochloride | 41.40 g | 5.00 mg | 41.40 g | 10.0 mg | 13.80 |
| Sodium metabisulfite | 4.14 g | 0.50 mg | 4.14 g | 1.00 mg | 1.38 |
| Copovidone, NF (KOLLIDON VA 64) | 3.97 g | 30.00 mg | 3.97 g | 60.00 mg | 82.81 |
| Sodium hydroxide | 248.40 g | 0.48 mg | 248.40 g | 0.96 mg | 1.32 |
| Polysorbate 80 | 0.60 g | 0.07 mg | 0.60 g | 0.14 mg | 0.19 |
| Sucralose | 1.49 g | 0.18 mg | 1.49 g | 0.36 mg | 0.50 |
| Purified Water | 7700.00 g | | 3700.00 g | | |
| **Total** | | 36.23 mg | | 72.46 mg | 100.0 |

The above ingredients were mixed with purified water according to the procedure outlined in Example 5 wherein the polysorbate 80 and sucralose were added to the apomorphine, sodium metabisulfate and copovidone solution prior to the addition of the sodium hydroxide solution. Empty aluminum blister cards were placed on stainless steel trays and the empty blister cavities were filled with the drug/polymer aqueous solution to the target fill weight of about 966 mg. The stainless steel trays comprising the filled blister cards were placed in a lyophilizer and lyophilized using the freeze cycle similar to Example 6. The target moisture level for the resulting tablets was 2-7%.

The lyophilized tablets in the blister cards were sealed with an aluminum lid and placed in an aluminum pouch with a DRI-CARD™ desiccant card purchased from Uline and the pouch was heat sealed. Other brands and types of desiccant materials can be used.

### EXAMPLE 13

Rapid orally disintegrable/dissolving tablet were prepared according to the procedure of Example 12 except the aluminum blister cards were replaced with polyvinylchloride (PVC) blister cards. The after lyophilization, the PVC blister cards were sealed with an aluminum lid and placed into aluminum pouches with either 0.5 g, 1.0 g (2 x 0.5) or 2.43g DRI-CARD™ desiccant cards purchased from Uline. The aluminum pouches were heat sealed. Samples of the heat sealed pouches were stored at 25°C and 60% relative humidity and at 40°C and 75% relative humidity. The results of the storage testing are as follows:

**For the 25°C/60% RH (0.5 g DRI-CARD™)**

| **Sample** | **RRT 0.36** | **RRT 0.42** | **RRT 0.69** | **RRT 0.83** | **RRT 0.96** | **RRT 1.22** | **RRT 1.25** | **RRT 1.36** | **RRT 1.36** | **RRT 1.41** | **RRT 1.58** | **RRT 1.99** |
|---|---|---|---|---|---|---|---|---|---|---|---|---|
| 10 mg (initial) | - | - | 0.09 | 0.05 | - | - | - | - | 0.04 | 0.03 | - | - |
| 10mg (2month) | - | - | 0.09 | 0.05 | - | - | - | - | - | 0.05 | - | - |
| 10 mg (4month) | - | - | 0.11 | 0.05 | - | - | - | - | 0.04 | 0.04 | - | - |
| 5 mg (initial) | - | - | 0.15 | 0.07 | - | - | - | - | 0.04 | 0.04 | - | - |
| 5mg (2month) | - | - | 0.14 | 0.07 | - | - | - | - | - | 0.03 | - | - |

| | | | | | | | | | | | | |
|---|---|---|---|---|---|---|---|---|---|---|---|---|
| "-" denotes impurities level less than 0.03% or not detectable. | | | | | | | | | | | | |

RRT refers to the relative retention time using a reversed-phase liquid chromatography (RP-HPLC) method, employing a C18 column, gradient, and ultraviolet (UV) detection at 273nm. Quantitation of impurities in the drug product is achieved by using a single point prepared Reference Standard of Apomorphine Hydrochloride. The equipment employed was a Waters HPLC apparatus equipped with UV/VIS detector and a PDA. The column was a Waters, SunFire C18, 3.5 µm, 4.6x150 mm. The analysis was run in the "gradient" mode with a column temperature of 30°C±5°C, sample temperature of 5°C±3°C and run time of 40 minutes. The mobile phase was:
A: water/trifluoroacetic acid (TFA)(100:0.1, v/v); and
B: acetonitrile:TFA (100:0.1, v/v) employed as follows:

| **Time (min)** | **Flow (mL/min)** | **%MP A** | **% MP B** |
|---|---|---|---|
| 0 | 1.0 | 90 | 10 |
| 30.0 | 1.0 | 75 | 25 |
| 30.1 | 1.0 | 10 | 90 |
| 32.0 | 1.0 | 10 | 90 |
| 32.1 | 1.0 | 90 | 10 |
| 40.0 | 1.0 | 90 | 10 |

It is believed that apomorphine orthoquinone is the impurity with an RRT of 0.69 and diethylapomorphine is the impurity with an RRT of 1.41. The identity of the other impurities is currently unknown.

**For the 40°C/75% RH (2 month time point)**

| **Sample** | **RRT 0.36** | **RRT 0.42** | **RRT 0.69** | **RRT 0.83** | **RRT 0.96** | **RRT 1.22** | **RRT 1.25** | **RRT 1.36** | **RRT 1.40** | **RRT 1.41** | **RRT 1.58** | **RRT 1.99** |
|---|---|---|---|---|---|---|---|---|---|---|---|---|
| 10 mg (0.5 g Dri-Card) | - | - | 0.09 | 0.08 | 0.04 | - | - | 0.03 | - | 0.25 | - | - |
| 10 mg (1.0 g Dri-Card) | - | - | 0.09 | 0.06 | 0.02 | - | - | - | - | 0.15 | - | - |
| 10 mg (2.4 g Dri-Card) | - | - | 0.08 | 0.06 | 0.03 | - | - | - | - | 0.21 | - | - |
| 10 mg (no Dri-Card) | 0.17 | 0.17 | 0.16 | 0.32 | 0.12 | 0.04 | 2.98 | - | 0.13 | 0.13 | 2.51 | 0.05 |

| | | | | | | | | | | | | |
|---|---|---|---|---|---|---|---|---|---|---|---|---|
| "-" denotes impurities level less than 0.03% or not detectable. | | | | | | | | | | | | |

**For the 40°C/75% RH**

| **Sample** | **RRT 0.26** | **RRT 0.60** | **RRT 0.69** | **RRT 0.83** | **RRT 0.96** | **RRT 1.25** | **RRT 1.35** | **RRT 1.42** | **RRT 1.51** | **RRT 1.59** | **RRT 1.79** | **RRT 1.97** |
|---|---|---|---|---|---|---|---|---|---|---|---|---|
| 10 mg (initial) | - | - | 0.05 | 0.03 | - | - | 0.04 | 0.04 | 0.02 | 0.25 | - | - |
| 10 mg (2 weeks) | 0.11 | 0.03 | 0.05 | 0.07 | 0.11 | - | 0.04 | 0.24 | 0.03 | 0.02 | - | 0.07 |
| 10 mg (1 month 0.5g Dri-Card) | - | - | 0.05 | 0.03 | - | - | 0.03 | 0.08 | - | - | - | - |

### EXAMPLE 14

The solubility of the apomorphine in the composition as described in Example 5, was compared to the solubility of USP grade apomorphine hydrochloride at varying pHs. The testing procedure was as followings:
1. Buffer solutions for pH 2.5, pH 3.0, pH 4.0, pH 5.0, pH 6.0, pH 6.2, pH 6.4, pH 6.6, pH 6.8, pH 7.0, pH 7.4, and pH 8.0 were prepared by mixing appropriate amount of 50 mM potassium phosphate monobasic (KH₂PO₄, and 50mM potassium phosphate dibasic (K₂HPO₄).
2. Apomorphine formulation samples (Example 5), ∼1.5g were weighed, and dispensed into vials containing into 20 mL of the phosphate buffer solution with pH ranging from pH 2.5 to 6.8, and ∼0.5g samples were weighted and dispensed into vials containing buffer solutions with pH ranging from pH 7.0 to pH 8.0. The samples were stirred for ∼18 hours before analysis.
3. Apomorphine hydrochloride samples, ∼0.4g were weighed and dispensed into vials containing 20 mL of the phosphate buffer solution with pH ranging from pH 2.5 to 5.0, and ∼0.2g samples were weighed and dispensed into vials containing buffer solutions with pH ranging from pH 6.0 to pH 8.0. The samples were stirred for ∼18hours before analysis.
4. Apomorphine base samples, ∼0.2g were weighed and dispensed into vials containing 20 mL of the phosphate buffer solution with pH ranging from pH 2.5 to 8.0. The samples were stirred for ∼18hours before analysis.
5. After stirring ∼2mL of the supernatant was obtained from each vial and centrifuge at 12000 RPM to precipitate un-dissolved drug.
6. After centrifugations, approximately 1mL of the supernatant was diluted with approximately 50 mL of water: methanol: H₃PO₄ (50:50:1.2 v/v) for HPLC analysis according to the procedure described in Example 13 above.

The solubility results are:

| pH | Apomorphine HCl | Example 5 | Apomorphine Free Base |
|---|---|---|---|
| 2.50 | 22.8 | >24.8 | 9.2 |
| 3.00 | 21.6 | >21.1 | 3.7 |
| 4.00 | 20.0 | >19.4 | 2.7 |
| 5.00 | 18.8 | >19.3 | 2.5 |
| 6.00 | 7.4 | >19.4 | 2.0 |
| 6.20 | 7.2 | >19.3 | 1.7 |
| 6.40 | 3.7 | >19.7 | 1.7 |
| 6.60 | 1.9 | >19.9 | 1.3 |
| 6.80 | 1.4 | >19.2 | 1.2 |
| 7.00 | 1.0 | 5.6 | 0.67 |
| 7.40 | 0.61 | 4.2 | 0.40 |
| 8.00 | 0.27 | 3.2 | 0.12 |

The above solubility data shows that the apomorphine present in the solid solution dosage form prepared in accordance with the present invention exhibits improved solubility compared to apomorphine hydrochloride, especially at pHs commonly encounter in a patient's oral cavity.

### EXAMPLE 15

The dissolution of the composition as described in Example 5 was compared to the dissolution of USP grade apomorphine hydrochloride. The following procedure was employed to obtain the dissolution profiles:
1. Simulated saliva media (aqueous phosphate buffered solution with a pH of 6.2± 1.0 was prepared and warmed to 37°C ± 0.5°C.
2. Samples of the composition of Example 5 and apomorphine hydrochloride were dispensed according to the table below into scintillation glass vials. The actual weight of the samples was recorded.

| | Target Weight of Sample | Average Weight | Simulated saliva Media |
|---|---|---|---|
| | (mg) | (n=12) | (mL) |
| Composition Example 5 | 35.0 | 35.38 | 5 |
| Apomorphine Hydrochloride, USP | 10.3 | 10.50 | 10 |

3. Approximately 5 mL or 10 mL of pre-warmed simulated saliva was added to the vials of step 2 according to the table above, and a stop watch was started. The vials with the media were gently swirled to allow the powder in the vials to go into solution.
4. At specific time points, samples of the media were withdrawn from the vials of step 3 and transferred into the barrel of a 10 mL syringe with a 0.22 mm filter attached to the tip of the syringe. The solution was pushed through the filter and collected for HPLC assay according to the procedure described in Example 13 above.
5. For sample time points longer than 5 minutes the vials of step 3 were placed in a 37°C incubator and shaken at 50 rpms until the collection time point. The results of the dissolution testing are shown in the following table:

| **Time** | **Example 5** | **Example 5** | **Apomorphine HCL** | **Apomorphine HCL** |
|---|---|---|---|---|
| **Initial** | 53.90% | 43.27% | 22.75% | 19.05% |
| **0.5 min** | 45.30% | 59.03 | 30.52% | 27.73% |
| **1 min** | 75.63% | 91.40% | 34.58% | 30.24% |
| **5 min** | 95.40% | 92.32% | 57.54% | 46.57% |
| **10 min** | 94.48% | 94.22% | 46.00% | 52.29% |
| **30 min** | 96.12% | 95.35% | 53.23% | 74.97% |

The above dissolution data shows that compositions prepared in accordance with the present invention exhibit a more rapid dissolution than apomorphine hydrochloride at pH commonly encountered in a patient's oral cavity. It is believed that this rapid dissolution results in an improved absorption of apomorphine from the patient's oral cavity because most, i.e., more than 75%, of the apomorphine in the compositions of the present invention is in solution within one minute and greater than 85% is in solution by 5 minutes. This rapid dissolution is important because an orally disintegrating dosage form typically remains in a patient's oral cavity for 5 minutes or less and most often 3 minutes or less.

### EXAMPLE 16

The composition as described in Example 5, was tested with a proton NMR apparatus (JOEL ECX-400NMR, sample were dissolved in Deuterated Methanol (DC3OD), and acquired at ambient temperature, with spectrometer operating at 400MHz. The resulting FIDs were transferred to a PC and processed using NUTS NMR Inc. 1H chemical shifts were referenced to TMS, 0 ppm). The NMR shift of the 1H ppm obtained from the composition of Example 5 was compared to the shifts obtained from known mixtures containing known amounts apomorphine free base and apomorphine hydrochloride. Based upon the comparison of the data from Example 5 and a calibration curve generated from the known sample mixtures, it is estimated that approximately 70% of the apomorphine in the composition of Example 5 is in the non-protonated form. The non-protonated form is the desired form for delivery to a patient's oral cavity because it highly permeable to the oral mucosa.

### EXAMPLE 17

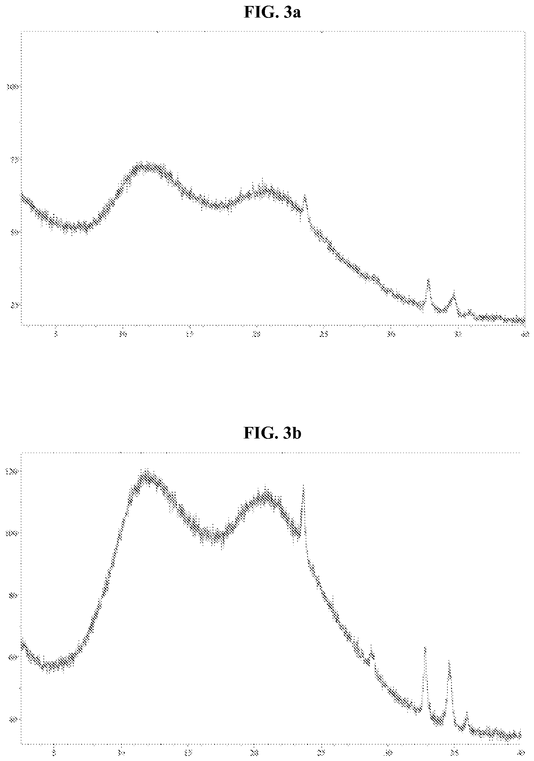

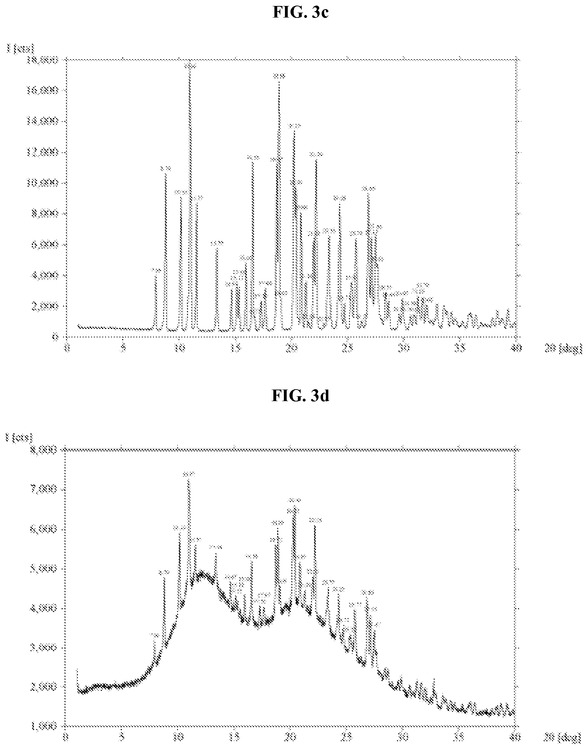

The X-ray powder diffraction (XRPD) pattern was obtained for the tablet prepared in Example 12 and the powder prepared in Example 5. XRPD patterns were also obtained from a sample of apomorphine hydrochloride and a physical mixture of the ingredients used to prepare the tablet of Example 12 but without the preparation of the solid solution. The XRPD pattern for the tablet is Example 12 is shown in Figure 3a. The XRPD pattern for the powder prepared in Example 5 is shown in Figure 3b. The XRPD pattern for the apomorphine hydrochloride is shown in Figure 3c and the XRPD pattern for the physical mixture of the ingredients used to prepare the tablet of Example 12 is shown in Figure 3d.

The XRPD patterns were collected with PANalytical X'Pert Pro MPD diffractometer using an incident beam of Cu radiation.

The XRPD pattern shows that the apomorphine present in the compositions of the present invention, tablets and powders, is in a substantially amorphous or non-crystalline form. It is believed that the 2θ peaks present in Figures 3a and 3b between 23-24 2θ, between 28-29 2θ, and between 32-36 2θ are due to sodium metabissulfite and not the apomorphine.

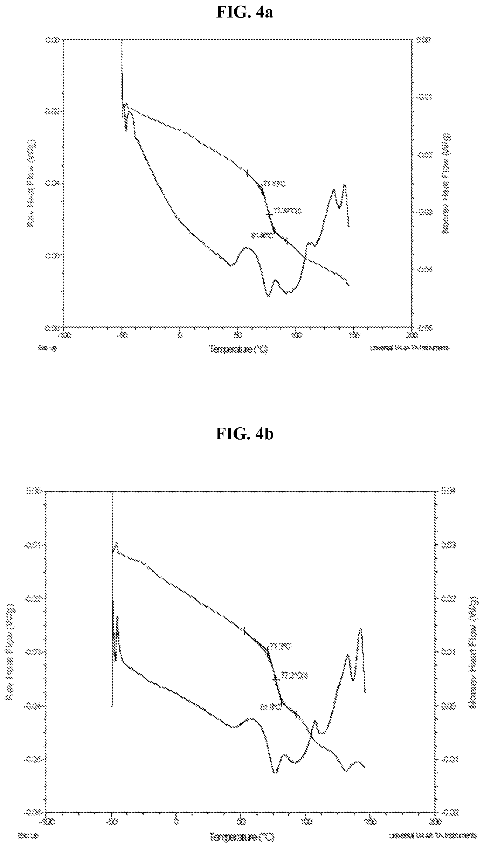

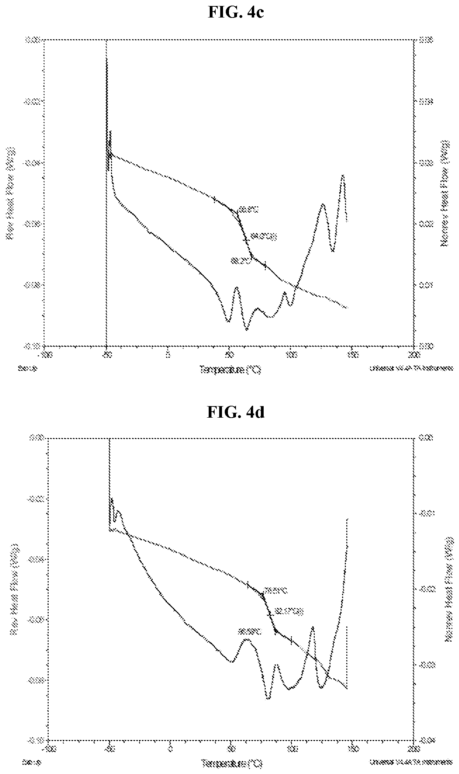

Modulated DSC data was also obtained for the tablet prepared in Example 12 and the powder prepared in Example 5. The modulated DSC thermogram for the tablet of Example 12 is shown in Figure 4a. The modulated DSC thermograms for the tablet of Example 12 stored at approximately 11% relative humidity and 22% relative humidity (in jars with saturated salt solutions) for about one week are shown in Figures 4b and 4c respectively. The modulated DSC thermogram for the powder of Example 5 is shown in Figure 4d.

The thermograms indicate that the tablet of Example 12 exhibits a glass transition temperature of about 77°C under normal conditions and after storage at 11% relative humidity for one week and 64°C after storage at 22% relative humidity for one week. The powder exhibits a glass transition temperature of about 82°C.

The thermograms were obtained with a TA Instruments Q2000 differential scanning calorimeter equipped with a refrigerated cooling system (RCS). Each sample was placed into an aluminum DSC pan and weighed. The pan was covered with a lid and sealed. Data obtained using a modulation amplitude of ±1.0°C and a 60 second period with an underlying heating rate of 2°C/minute from -80°C to 50°C or from -50°C to 150°C. The glass transition temperatures were determined from the inflection point of the step change in the reversing heat flow versus temperature curve.

### EXAMPLE 18

The powder formulation of Example 5 was administered to healthy human volunteer subjects between the ages of 18 and 45 years, in a single-center, open-label, randomized, single-dose, randomized, four treatment, crossover study. Twenty subjects were enrolled. Each subject received a single dose of the following treatments under fasting conditions:
Treatment 1, approximately 0.25 mL of a solution containing 5.0 mg of apomorphine hydrochloride hemihydrate (4.86 mg apomorphine hydrochloride) was placed in the sublingual region via a syringe;
Treatment 2, approximately 0.25 mL of a solution containing 5.0 mg of apomorphine hydrochloride hemihydrate (4.86 mg apomorphine hydrochloride) was placed in the sublingual region via a pump spray;
Treatment 3, approximately 3.42 mg of the powder prepared in Example 5 containing 5.0 mg of apomorphine hydrochloride hemihydrate (4.86 mg apomorphine hydrochloride) was placed in the sublingual; and
Treatment 4, Apomorphine injection, approximately 0.2 mL of a commercially available apomorphine injection (containing 9.72 mg of apomorphine hydrochloride/mL for a dose of about 1.94 mg apomorphine hydrochloride).

The subjects were instructed to swallow saliva immediately prior to the sublingual administration and to hold the composition in the sublingual region for at least 30 seconds without swallowing. The subjects were not allowed to expectorate, drink, or rinse the mouth for at least one hour post dose.

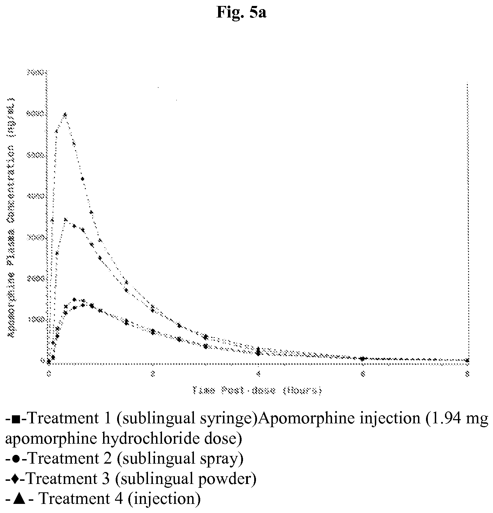

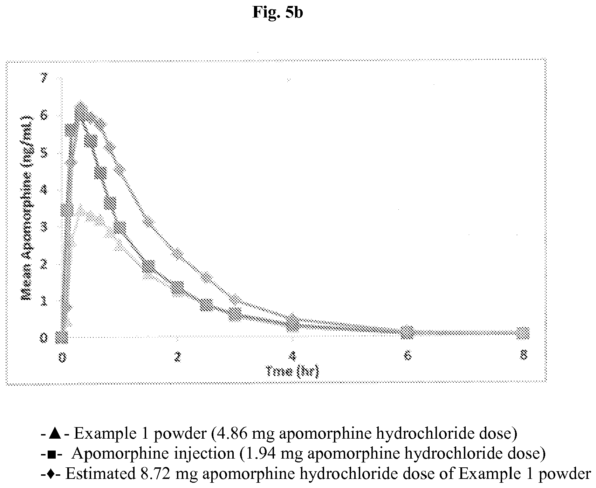

Plasma samples were collected within one hour predose and postdose at 5, 10, 20, 30, 40 and 50 minutes and at 1, 1.5, 2, 2.5, 3, 4, 6, and 8 hours. The plasma samples were obtained by collecting approximately 6 mL of blood in a K₂-EDTA treated tubes at each time point. The plasma was fractionated from the blood sample within 30 minutes of collection by centrifugation for about 10 minutes at 3000 rpm. The plasma samples were transferred via a pipette into L-ascorbic acid pretreated chilled polypropylene tubes and stored at -70°C until shipment for analysis. The resultant plasma samples were analyzed for apomorphine concentrations by a validated liquid chromatography-tandem mass spectrometry (HPLC-MS/MS) method. The plasma profile for the four treatments is shown in Figure 5 a. The plasma profile for the apomorphine injection and administration of the powder composition of Example 5 are shown in Figure 5b along with an estimated plasma profile curve for a theoretical administration of the powder composition of Example 5 at a dose of 8.72 mg of apomorphine hydrochloride (assuming dose proportionality). The mean (CV%) pharmacokinetic parameters for the seventeen (17) subjects that completed all portions of the study are:

| | **Treatment 1** | **Treatment 2** | **Treatment 3** | **Treatment 4** |
|---|---|---|---|---|
| | Sublingual syringe | Sublingual spray | Sublingual powder | injection |
| **Cₘₐₓ** | 1652.66 | 1531.24 | 3758.60 | 7601.93 |
| (ng/mL) | (36%) | (35%) | (35%) | (65%) |
| **Tₘₐₓ** | 0.50 | 0.67 | 0.33 | 0.33 |
| (hr) | (0.33-1.50) | (0.33-1.00) | (0.17-0.67) | (0.08-0.67) |
| **AUC₀₋ₜ** | 3386 | 3129 | 6286 | 8488 |
| (hr*ng/mL) | (36%) | (41%) | (34%) | (23%) |
| **AUC_{0-inf}** | 3460 | 3192 | 6361 | 8577 |
| (hr*ng/mL) | (35%) | (41%) | (33%) | (23%) |
| **T_{1/2}** | 1.32 | 1.31 | 1.09 | 1.22 |
| (hr) | (1.02-2.77) | (0.94-1.72) | (0.89-2.93) | (1.02-2.08) |

The invention illustratively described herein suitably may be practiced in the absence of any element or elements, limitation or limitations which is not specifically disclosed herein. Thus, for example, in each instance herein any of the terms "comprising," "consisting essentially of' and "consisting of' may be replaced with either of the other two terms.

## Claims

1. A solid dosage form for oral mucosal delivery of 0.1 to 100 mg of apomorphine comprising apomorphine and polymer solid solution wherein the apomorphine and polymer solid solution comprises
(i) 50 wt% or less of the apomorphine wherein the apomorphine is present in the apomorphine and polymer solid solution as an apomorphine acid addition salt and an apomorphine free base;
(ii) 50 wt% or more of a water soluble polymer that exhibits a viscosity of less than 500 mPa s when a 10% aqueous solution is prepared;
and the apomorphine and polymer solid solution dissolves within 5 minutes or less when placed in simulated saliva with a pH of 6.2 ± 1.0 and at a temperature of 37 ± 0.5°C, and wherein the apomorphine in the apomorphine and polymer solid solution is in at least 50% amorphous form.

2. The solid dosage form of claim 1 further comprising an antioxidant in an amount of from 0.05 wt% to 10 wt%.

3. The solid dosage form of claim 1 wherein the apomorphine and polymer solid solution dissolves within 2.5 minutes or less, or within 1 minute or less, when placed in simulated saliva with a pH of 6.2 ± 1.0 and at a temperature of 37 ± 0.5°C.

4. The solid oral dosage form of claim 1 wherein the water soluble polymer (ii) is selected from a group consisting of povidone, copovidone, hydroxypropyl methylcellulose, hydroxypropyl cellulose, low-substituted hydroxypropyl cellulose, polyvinyl alcohols, polyvinyl alcohol-polyethylene glycol copolymers, and mixtures thereof.

5. The solid oral dosage form of claim 2 wherein the antioxidant is selected from a group consisting of ascorbic acid, ascorbyl palmitate, butylated hydroxyanisole, butylated hydroxytoluene, hypophosphorous acid, monothioglycerol, potassium metabisulfate, propyl gallate, sodium bisulfite, sodium formaldehyde sulfoxylate, sodium metabisulfate, sodium sulfate, sodium thiosulfate, sodium dioxide, tocopherol, and mixtures thereof.

6. The solid oral dosage form of of any preceding claim wherein the apomorphine and polymer solid solution comprises 0.01 wt% to 10 wt% of a solubilizer.

7. The solid oral dosage form of claim 6 wherein the solubilizer is selected from the group consisting of an ionic surfactant, a nonionic surfactant, or mixtures thereof or from the group consisting of polysorbates, derivatives of tocopherol, poloxamers, monoglycerides, diglycerides, fatty acids, fatty alcohols, and mixtures thereof.

8. The solid dosage form of claim 1 wherein the solid dosage form is a tablet shaped for administration to a patient's buccal, sublingual or gingival regions, further comprising at least one excipient selected from the group consisting of fillers, binders, disintegrants, sweeteners, flavoring agents, pH adjusting agents, solubilizing agent, lubricants, glidants and mixtures thereof.

9. The solid dosage form of claim 1 wherein the apomorphine and polymer solid solution comprises:
(i) 2 wt% to 40 wt% of the apomorphine wherein the apomorphine is present in the apomorphine and polymer solid solution as an apomorphine acid addition salt and an apomorphine free base;
(ii) 50 wt% to 95 wt% of a water soluble polymer that exhibits a viscosity of less than 500 mPa s when a 10% aqueous solution is prepared.

10. The solid oral dosage form of any preceding claim wherein the apomorphine acid addition salt is apomorphine hydrochloride.

11. The solid oral dosage form of claim 6, 9 and 10 wherein the solubilizer is selected from a group consisting of polysorbates, derivatives of tocopherol, poloxamers, monoglycerides, diglycerides, fatty acids, fatty alcohols, and mixtures thereof.

12. The solid oral dosage form of claims 9, 10 or 11, wherein the apomorphine hydrochloride is combined with 0.50 moles to 0.95 moles of a base during formation of the apomorphine and polymer solid solution to create the apomorphine free base.

13. The solid oral dosage form of claims 9, 10 or 11, wherein the apomorphine hydrochloride is combined with 0.60 moles to 0.92 moles of a base during formation of the apomorphine and polymer solid solution to create the apomorphine free base.

14. The solid oral dosage form of claims 9, 10 or 11, wherein the apomorphine hydrochloride is combined with 0.65 moles to 0.90 moles of a base during formation of the apomorphine and polymer solid solution to create the apomorphine free base.

15. The solid oral dosage form of any of the preceding claims for use in the treatment of Parkinson's disease by oral cavity administration of an effective amount of the rapidly disintegrating and dissolving solid dosage form.

## Patentansprüche

1. Eine feste Dosierform für orale Schleimhautanlieferung von 0,1 bis 100 mg Apomorphin, umfassend eine feste Lösung von Apomorphin und einem Polymer, wobei die feste Lösung von Apomorphin und einem Polymer Folgendes umfasst
(i) 50 Gew.-% oder weniger des Apomorphins, wobei das Apomorphin in der festen Lösung von Apormorphin und einem Polymer als Apomorphin-Säureadditionssalz und eine freie Apomorphin-Base vorhanden ist;
(ii) 50 Gew.-% oder mehr eines wasserlöslichen Polymers, das eine Viskosität von weniger als 500 mPa s aufweist, wenn eine 10%ige wässrige Lösung hergestellt wird;
und wobei sich die feste Lösung von Apomorphin und einem Polymer innerhalb von 5 min oder weniger auflöst, wenn diese in simuliertem Speichel mit einem pH von 6,2 ± 1,0 und einer Temperatur von 37 ± 0,5 °C platziert wird, und wobei das Apomorphin in der festen Lösung von Apomorphin und einem Polymer zumindest zu 50 % in amorpher Form vorliegt.

2. Die feste Dosierform nach Anspruch 1, ferner umfassend ein Antioxidans in einer Menge von 0,05 Gew.-% bis 10 Gew.-%.

3. Die feste Dosierform nach Anspruch 1, wobei sich die feste Lösung von Apomorphin und einem Polymer innerhalb von 2,5 min oder weniger, oder innerhalb von 1 min oder weniger auflöst, wenn diese in simuliertem Speichel mit einem pH von 6,2 ± 1,0 und einer Temperatur von 37 ± 0,5 °C platziert wird.

4. Die feste orale Dosierform nach Anspruch 1, wobei das wasserlösliche Polymer (ii) aus einer Gruppe, bestehend aus Povidon, Copovidon, Hydroxypropylmethylcellulose, Hydroxypropylcellulose, niedersubstituierter Hydroxypropylcellulose, Polyvinylalkoholen, Polyvinylalkohol-Polyethylenglykol-Copolymeren und Gemischen davon, ausgewählt ist.

5. Die feste orale Dosierform nach Anspruch 2, wobei das Antioxidans aus einer Gruppe, bestehend aus Ascorbinsäure, Ascorbylpalmitat, butyliertem Hydroxyanisol, butyliertem Hydroxytoluol, Hypophosphorsäure, Monothioglycerol, Kaliummetabisulfat, Propylgallat, Natriumbisulfit, Natriumformaldehydsulfoxylat, Natriummetabisulfat, Natriumsulfat, Natriumthiosulfat, Natriumdioxid, Tocopherol und Gemischen davon, ausgewählt ist.

6. Die feste orale Dosierform nach einem vorstehenden Anspruch, wobei die feste Lösung von Apomorphin und einem Polymer 0,01 Gew.-% bis 10 Gew.-% eines Lösungsvermittlers umfasst.

7. Die feste orale Dosierform nach Anspruch 6, wobei der Lösungsvermittler aus der Gruppe, bestehend aus einem ionischen Tensid, einem nichtionischen Tensid oder Gemischen davon, oder aus der Gruppe, bestehend aus Polysorbaten, Derivaten von Tocopherol, Poloxameren, Monoglyceriden, Diglyceriden, Fettsäuren, Fettalkoholen und Gemischen davon, ausgewählt ist.

8. Die feste Dosierform nach Anspruch 1, wobei die feste Dosierform eine Tablette ist, die für die Verabreichung an eine bukkale, sublinguale oder gingivale Region eines Patienten geformt ist, ferner umfassend mindestens einen Exzipienten, der aus der Gruppe, bestehend aus Füllern, Bindemitteln, Auflösungsmitteln, Süßstoffen, Geschmacksstoffen, pH-Anpassungsmitteln, Lösungsvermittlern, Schmiermitteln, Gleitmitteln und Gemischen davon, ausgewählt ist.

9. Die feste Dosierform nach Anspruch 1, wobei die feste Lösung von Apomorphin und einem Polymer Folgendes umfasst:
(i) 2 Gew.-% bis 40 Gew.-% des Apomorphins, wobei das Apomorphin in der festen Lösung von Apormorphin und einem Polymer als Apomorphin-Säureadditionssalz und eine freie Apomorphin-Base vorhanden ist;
(ii) 50 Gew.-% bis 95 Gew.-% eines wasserlöslichen Polymers, das eine Viskosität von weniger als 500 mPa s aufweist, wenn eine 10%ige wässrige Lösung hergestellt wird.

10. Die feste orale Dosierform nach einem der vorstehenden Ansprüche, wobei das Apomorphin-Säureadditionssalz Apomorphinhydrochlorid ist.

11. Die feste orale Dosierform nach Anspruch 6, 9 und 10, wobei der Lösungsvermittler aus einer Gruppe, bestehend aus Polysorbaten, Derivaten von Tocopherol, Poloxameren, Monoglyceriden, Diglyceriden, Fettsäuren, Fettalkoholen und Gemischen davon, ausgewählt ist.

12. Die feste orale Dosierform nach Anspruch 9, 10 oder 11, wobei das Apomorphinhydrochlorid während der Bildung der festen Lösung von Apomorphin und einem Polymer mit 0,50 Mol bis 0,95 Mol einer Base vereinigt wird, um die freie Apomorphin-Base zu erzeugen.

13. Die feste orale Dosierform nach Anspruch 9, 10 oder 11, wobei das Apomorphinhydrochlorid während der Bildung der festen Lösung von Apomorphin und einem Polymer mit 0,60 Mol bis 0,92 Mol einer Base vereinigt wird, um die freie Apomorphin-Base zu erzeugen.

14. Die feste orale Dosierform nach Anspruch 9, 10 oder 11, wobei das Apomorphinhydrochlorid während der Bildung der festen Lösung von Apomorphin und einem Polymer mit 0,65 Mol bis 0,90 Mol einer Base vereinigt wird, um die freie Apomorphin-Base zu erzeugen.

15. Die feste orale Dosierform nach einem der vorstehenden Ansprüche zur Verwendung bei der Behandlung von Morbus Parkinson durch Verabreichung einer wirksamen Menge der rasch zerfallenden und sich rasch auflösenden festen Dosierform an die Mundhöhle.

## Revendications

1. Forme posologique solide, pour l'administration à travers la muqueuse buccale, de 0,1 à 100 mg d'apomorphine, comprenant une solution solide d'apomorphine et de polymère, dans laquelle la solution solide d'apomorphine et de polymère comprend
(i) 50 % en poids ou moins d'apomorphine, dans laquelle l'apomorphine est présente dans la solution solide d'apomorphine et de polymère sous la forme d'un sel d'addition d'acide d'apomorphine et d'une base libre d'apomorphine;
(ii) 50 % en poids ou plus d'un polymère soluble dans l'eau qui présente une viscosité inférieure à 500 mPa.s lors de la préparation d'une solution aqueuse à 10 %;
et la solution solide d'apomorphine et de polymère se dissout en 5 minutes ou moins, lorsque placée dans de la salive simulée, avec un pH de 6,2 ± 1,0 et à une température de 37 ± 0,5 °C, et dans laquelle l'apomorphine présente dans la solution solide d'apomorphine et de polymère est à au moins 50 % de forme amorphe.

2. Forme posologique solide selon la revendication 1, qui comprend en outre un antioxydant en une quantité de 0,05 % en poids à 10 % en poids.

3. Forme posologique solide selon la revendication 1, dans laquelle la solution solide d'apomorphine et de polymère se dissout en 2,5 minutes ou moins, ou en 1 minute ou moins, lorsque placée dans une salive simulée avec un pH de 6,2 ± 1,0 et à une température de 37 ± 0,5 °C.

4. Forme posologique solide orale selon la revendication 1, dans laquelle le polymère soluble dans l'eau (ii) est choisi dans un groupe comprenant la povidone, la copovidone, l'hydroxypropylméthylcellulose, l'hydroxypropylcellulose, l'hydroxypropylcellulose faiblement substituée, les alcools polyvinyliques, les copolymères d'alcool polyvinylique et de glycol polyéthylénique, et leurs mélanges.

5. Forme posologique solide orale selon la revendication 2, dans laquelle l'antioxydant est choisi dans un groupe comprenant l'acide ascorbique, le palmitate d'ascorbyle, l'hydroxyanisole butylé, l'hydroxytoluène butylé, l'acide hypophosphoreux, le monothioglycérol, le métabisulfate de potassium, le propyl gallate, le bisulfite de sodium, le sulfoxylate de formaldéhyde de sodium, le métabisulfate de sodium, le sulfate de sodium, le thiosulfate de sodium, le dioxyde de sodium, le tocophérol, et leurs mélanges.

6. Forme posologique solide orale selon l'une quelconque des revendications précédentes, dans laquelle la solution solide d'apomorphine et de polymère comprend 0,01 % en poids à 10 % en poids d'un solubilisant.

7. Forme posologique solide orale selon la revendication 6, dans laquelle le solubilisant est choisi dans le groupe comprenant un tensioactif ionique, un tensioactif non ionique ou leurs mélanges ou dans le groupe comprenant les polysorbates, les dérivés du tocophérol, les poloxamères, les monoglycérides, les diglycérides, les acides gras, les alcools gras et leurs mélanges.

8. Forme posologique solide selon la revendication 1, dans laquelle la forme posologique solide est un comprimé formé pour l'administration dans les régions buccale, sublinguale ou gingivale d'un patient, qui comprend en outre au moins un excipient choisi dans le groupe comprenant les charges, les liants, les délitants, les édulcorants, les aromatisants, les agents d'ajustement du pH, les agents solubilisants, les lubrifiants, les glissants, et leurs mélanges.

9. Forme posologique solide selon la revendication 1, dans laquelle la solution solide d'apomorphine et de polymère comprend :
(i) 2 % en poids à 40 % en poids d'apomorphine, dans laquelle l'apomorphine est présente dans la solution solide d'apomorphine et de polymère sous la forme d'un sel d'addition d'acide d'apomorphine et d'une base libre d' apomorphine;
(ii) 50 % en poids à 95 % en poids d'un polymère soluble dans l'eau qui présente une viscosité inférieure à 500 mPa.s lors de la préparation d'une solution aqueuse à 10 %.

10. Forme posologique solide orale selon l'une quelconque des revendications précédentes, dans laquelle le sel d'addition d'acide d'apomorphine est le chlorhydrate d'apomorphine.

11. Forme posologique solide orale selon les revendications 6, 9 et 10, dans laquelle l'agent solubilisant est choisi dans un groupe comprenant les polysorbates, les dérivés de tocophérol, les poloxamères, les monoglycérides, les diglycérides, les acides gras, les alcools gras, et leurs mélanges.

12. Forme posologique solide orale selon les revendications 9, 10 ou 11, dans laquelle le chlorhydrate d'apomorphine est combiné à 0,50 à 0,95 mole d'une base pendant la formation de la solution solide d'apomorphine et de polymère pour créer une base libre d'apomorphine.

13. Forme posologique solide orale selon les revendications 9, 10 ou 11, dans laquelle le chlorhydrate d'apomorphine est combiné à 0,60 à 0,92 mole d'une base pendant la formation de la solution solide d'apomorphine et de polymère pour créer une base libre d'apomorphine.

14. Forme posologique solide orale selon les revendications 9, 10 ou 11, dans laquelle le chlorhydrate d'apomorphine est combiné à 0,65 à 0,90 mole d'une base pendant la formation de la solution solide d'apomorphine et de polymère pour créer une base libre d'apomorphine.

15. Forme posologique solide orale selon l'une quelconque des revendications précédentes, utilisée dans le traitement de la maladie de Parkinson par administration dans la cavité buccale d'une quantité efficace de la forme posologique solide à dissolution et désintégration rapides.
